(19) Europäisches Patentamt / European Patent Office / Office européen des brevets

(11) **EP 3 978 612 B1**

(12) **EUROPEAN PATENT SPECIFICATION**

(45) Date of publication and mention of the grant of the patent:
**02.07.2025 Bulletin 2025/27**

(21) Application number: **20814172.1**

(22) Date of filing: **29.05.2020**

(51) International Patent Classification (IPC):
**G01N 33/58** (2006.01)     **C12N 15/81** (2006.01)
**C12P 21/00** (2006.01)

(52) Cooperative Patent Classification (CPC):
**C12N 15/815; C07K 14/43595; C12P 21/02; G01N 33/582; G01N 33/68;** C07K 2319/60

(86) International application number:
**PCT/CN2020/093500**

(87) International publication number:
**WO 2020/239111 (03.12.2020 Gazette 2020/49)**

(54) **METHOD FOR QUANTITATIVE CO-EXPRESSING MULTIPLE PROTEINS IN VITRO AND APPLICATION THEREOF**

VERFAHREN ZUR QUANTITATIVEN CO-EXPRESSION MEHRERER PROTEINE IN VITRO UND SEINE ANWENDUNG

PROCÉDÉ DE CO-EXPRESSION QUANTITATIVE DE MULTIPLES PROTÉINES IN VITRO ET APPLICATION ASSOCIÉE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: **30.05.2019 CN 201910460987**

(43) Date of publication of application:
**06.04.2022 Bulletin 2022/14**

(73) Proprietor: **Kangma-Healthcode (Shanghai) Biotech Co., Ltd.**
**Shanghai 201321 (CN)**

(72) Inventors:
• **GUO, Min**
  **Shanghai 201321 (CN)**
• **YU, Xue**
  **Shanghai 201321 (CN)**

(74) Representative: **Cabinet Chaillot**
**16/20, avenue de l'Agent Sarre**
**B.P. 74**
**92703 Colombes Cedex (FR)**

(56) References cited:
CN-A- 105 039 379     US-A1- 2002 106 719

• JIN-HO AHN ET AL: "Tuning the expression level of recombinant proteins by modulating mRNA stability in a cell-free protein synthesis system", BIOTECHNOLOGY AND BIOENGINEERING, JOHN WILEY, HOBOKEN, USA, vol. 101, no. 2, 7 March 2008 (2008-03-07), pages 422 - 427, XP071134047, ISSN: 0006-3592, DOI: 10.1002/BIT.21884

• JIAN LI ET AL: "Establishing a high yielding streptomyces-based cell-free protein synthesis system", BIOTECHNOLOGY AND BIOENGINEERING, JOHN WILEY, HOBOKEN, USA, vol. 114, no. 6, 23 February 2017 (2017-02-23), pages 1343 - 1353, XP071153358, ISSN: 0006-3592, DOI: 10.1002/BIT.26253

• PARK YU JIN ET AL: "Assessing translational efficiency by a reporter protein co-expressed in a cell-free synthesis system", ANALYTICAL BIOCHEMISTRY, ACADEMIC PRESS, AMSTERDAM, NL, vol. 518, 29 November 2016 (2016-11-29), pages 139 - 142, XP029850671, ISSN: 0003-2697, DOI: 10.1016/J.AB.2016.11.019

**EP 3 978 612 B1**

**(Cont. next page)**

- GARENNE DAVID ET AL: "Cell-free transcription-translation: engineering biology from the nanometer to the millimeter scale", CURRENT OPINION IN BIOTECHNOLOGY, LONDON, GB, vol. 58, 3 November 2018 (2018-11-03), pages 19 - 27, XP085798861, ISSN: 0958-1669, [retrieved on 20181103], DOI: 10.1016/J.COPBIO.2018.10.007
- HU, YUAN ET AL.: "Synthesis of soluble proinsulin in a cell-free protein synthesis system", CHINESE JOURNAL OF BIOTECHNOLOGY, vol. 33, no. 3, 25 March 2017 (2017-03-25), pages 467 - 477, XP055761981, ISSN: 1000-3061
- KIM, DM ET AL.: "Development of a rapid and productive cell free protein synthesis system", BIOTECHNOLOGY AND BIOPROCESS ENGINEERING, vol. 11, no. 3, 30 June 2006 (2006-06-30), pages 235 - 239, XP055761985, ISSN: 1226-8372

## Description

The present application claims priority to Chinese Patent Application No. CN 201910460987.8, filed on May 30, 2019.

## BACKGROUND OF THE INVENTION

### 1. Technical Field

The present invention relates to the field of biotechnology, and more particularly, to a method for quantitatively co-expressing multiple proteins *in vitro* and application thereof.

### 2. Description of the Related Art

Fluorescent proteins have been widely used in many research fieldsof biology. Fluorescent protein-based molecular probes and labeling methods have become important research tools for studying biological macromolecules or cell functions through dynamic imaging in living cells or *in vivo.* Since the gene for green fluorescent protein was first cloned from jellyfish in 1992, lots of new fluorescent proteins have been cloned from many marine species and new mutants have been obtained from the modification of fluorescent proteins. Those new fluorescent proteins and mutants can"light up"biomolecules or cells, and exhibit the biomolecules activity, thereby helping us reveal the activity law and nature of these molecules or cells. The spectra of the reported fluorescent proteins are distributed throughout the visible region. Those fluorescent proteins are widely used in such areas as gene expression and regulation, protein spatial positioning and transport, protein folding, signal transduction, protease activity analysis, and biomolecular interaction. The discovery and application of the fluorescent proteins provide a powerful research means for the study of modern biology.

Due to the advances in biotechnology, synthetic biologists are able to quickly and reliably transform biological systems and design and produce biological macromolecules, especially to design and produce protein macromolecules with biological functions. In the development of synthetic biology in the past few years, proteins are mainly engineered inside cells with cells as hosts, but it istime-consuming and difficultto engineer inside the cells. This is because the process of cell growth and adaptability is usually inconsistent with the goal of engineering design and the modification of biological components is greatly limiteddue to the complexity of living cell systems, the difficulty of standardization of genetic elements and the barrier created by cell membrane. In front of those limitations, people try to explore new development directions, which promote the development of a new discipline of engineering technology, that is, cell-free synthetic biology. The cell-free synthetic biology involves an emerging technology-- cell-free synthesis system, also known as *in vitro* synthesis system, comprising *in vitro* transcription and translation. In the cell-free protein synthesis system, exogenous target mRNA or DNA serve as the template for protein synthesis, and substrates and transcription and translation-related protein factors required for protein synthesis are manually controlled to synthesize the target proteins. The cell-free protein synthesis system eliminates the steps of plasmid construction, transformation, cell culture, cell collection, and fragmentation to synthesize and express proteins. Therefore, it is a fast, time-saving and convenient way to express protein.

Due to the properties of fluorescent proteins, the fluorescent proteins play an important and irreplaceable role in biological research. How to quantitatively co-express multiple proteins *in vitro* is an important technical problemfacing at present. After research and retrieval of literatures, we did not find any reports of quantitatively co-expressing multiple proteins in the same reaction system using *in vitro* cell-free protein synthesis technology. After a lot of experiments and researches on the present invention, we find how to quantitatively co-express multiple fluorescent proteins by adjusting the volume or concentration ratio of template DNA. It can be used for high-efficiency fluorescent indicator molecules, the research and development of fluorescently-labeled cells or organisms for various researches or other purposes, and the like.

## SUMMARY OF THE INVENTION

A method for quantitatively co-expressing multiple proteins *in vitro* is disclosed in the present invention. In this method, it is possible to quantitatively co-express multiple proteins in the same reaction system according to the concentration and dosage proportional relation of the template (preferably DNA template) of target protein.

According to the first aspect, the present invention provides a method for quantitatively co-expressing multiple proteins *in vitro,* comprising the steps of:

(1) Establishing a standard curve.

Establishing a standard curve of the relationship between concentration of each of standard proteins and respective luminescence value.

In Step (1), according to the types of multiple target proteins to be co-expressed, a standard protein corresponding to each target protein is provided; each of the multiple target proteins is independently a protein with a luminous function, and the multiple target proteins can be distinguished from each other based on luminescence properties, and can be detected separately "without mutual interference", thus, quantitative detection of each target protein can be achieved.

In particular, the standard protein in Step (1) is a standard sample of the target proteins. It can be obtained by separation and purification of the target proteins after synthesis thereof, or it is possible to purchase commercially available analytical pure products.

(2) Respectively creating vectors containing different target protein genes, respectively for expressing multiple different target proteins.

The expression "vectors containing target protein genes" means that the vectors contain encoding sequences of the target proteins, and they can also be referred to as "vectors of the target proteins". For example, "vectors encoding GFP protein" can be simply referred to as "GFP vectors".

In this step, separate vectors containing target protein genes are created to express each of the target proteins respectively. For each of the target proteins, a separate vector containing its encoding sequence is created, and the target proteins are independently expressed in a co-expression system through their respective separate vectors.

(3) Establishing an equation of quantitative relationship between concentration percentage of each of the target proteins and concentration percentage of a corresponding vector.

The separate vectors containing the target protein genes in Step (2) are added to an *in vitro* cell-free protein synthesis system at different concentration ratios for protein synthesis reaction *in vitro,* that is, incubation reaction, so as to synthesize the multiple target proteins; after a specified reaction time, a luminescence value for each target protein in a reaction solution is obtained; concentration of each target protein product is obtained according to the standard curve shown in Step (1), and an equation of quantitative relationship between concentration percentage of each target protein product and concentration percentage of a corresponding vector is obtained by fitting; in the *in vitro* cell-free protein synthesis system, the total concentration of the vectors remains the same.

When the separate vectors containing the target protein genes are addedat different concentration ratios, in the *in vitro* cell-free protein synthesis system, the total vector concentration during multiple *in vitro* protein reactions remains the same.

The total vector concentration refers to a sum of the vectors' concentration of all the target proteins in the *in vitro* cell-free protein synthesis system.

(4) Calculating concentration and concentration ratio of the vectors required for quantitatively co-expressing the multiple proteins.

The productconcentration ratio relationship (such as the mass concentration ratiorelationship) of the multiple target proteins to be achieved is set as the target concentration ratio of the multiple target proteins; the concentration and concentration ratio of the vector for each of the multiple target proteins to be expressed are calculated by using the equation established in Step (3).

(5) Quantitatively co-expressing the multiple proteins.

According to the required concentration and/or concentration ratio of each target proteinvector obtained in Step (4), acorresponding amount of the separate vector of each target protein is added to the *in vitro* cell-free protein synthesis system as described in Step (3), and the multiple target proteinsco-expressed are obtained after being reacted for the specific period of time defined in Step (3).

The multiple target proteins are each independently luminescent protein or fusion protein carrying a luminescent label.

When the concentration of each target protein separate vector in each mother solution is the same, the dosage can also be controlled simply by volume or volume ratio. Examples are as follows:

A method for quantitatively co-expressing multiple proteins in vitro, comprising the steps of: (1) establishing a standard curve: establishing standard curve of protein concentration-luminescence intensity relationship for each co-expressed protein using corresponding standard protein; (2) creating separate vectors and mother solutions thereof containing each target protein gene for expressing each target protein respectively, wherein the concentration of the separate vectors of each target protein in each mother solution is the same; (3) establishing an equation of quantitative relationship between concentration percentage of each target protein and volume percentage of a corresponding vector; wherein the separate vectors containing the target protein genes in Step (2) are added to the in vitro cell-free protein synthesis system at different volume ratios for protein synthesis reaction in vitro; after a specified reaction time, a luminescence value for each target protein in a reaction solution is obtained; the concentration of each target protein product is calculated according to the standard curve shown in Step (1), and the equation of quantitative relationship between concentration percentage of each target protein and the volume percentage of the corresponding vector is obtained by fitting; in the in vitro cell-free protein synthesis system, the total concentration of the vectors remains the same; (4) calculating the vector volume, and corresponding volume ratio required for quantitatively co-expressing the multiple proteins; wherein, according to target concentration ratio relationship of the multiple target proteins to be expressed, the volume and volume ratio of the vector required for each of the multiple target proteins to be expressed are calculated by using the equation established in Step (3); (5) quantitatively co-expressing the multiple proteins; wherein, according to the required volume and volume ratio for each target protein vector obtained in Step (4), a corresponding amount of the separate vector of each target protein is added to the in vitro cell-free protein synthesis system as described in Step (3), and the multiple target proteins co-expressed are obtained after being reacted for the specific period of time defined in Step (3); wherein the multiple target proteins are each independently luminescent protein or fusion protein carrying a luminescent label.

The *in vitro* cell-free protein synthesis system comprises at least the components required for protein synthesis except for the template. The *in vitro* cell-free protein synthesis system may or may not comprise the template. The *in vitro* cell-free protein synthesis system can also be prepared in a laboratory, or can be a commercially available product.

As one of the preferred embodiments, the *in vitro* cell-free protein synthesis system comprises:

(a) yeast cell extract;
(b) polyethylene glycol;
(c) optional exogenous sucrose; and
(d) optional solvent, wherein the solvent is water or aqueous solvent.

Wherein, the yeast cell may be derived from wild-type cells or genetically modified cells.

Wherein, the wording "optional" in components (c) and (d) refers to be dispensable, and components (c) and (d) are each independently dispensable.

In a preferred embodiment, the *in vitro* cell-free protein synthesis system comprises: yeast cell extract, trihydroxymethylaminomethane (Tris), potassium acetate, magnesium acetate, nucleoside triphosphate mixture (NTPs), amino acid mixture, potassium phosphate, amylase, polyethylene glycol, maltodextrin, etc.

In another preferred embodiment, the *in vitro* cell-free protein synthesis system provided in the present invention comprises: yeast cell extract, Tris, potassium acetate, magnesium acetate, nucleoside triphosphate mixture (NTPs), amino acid mixture, potassium phosphate, amylase, polyethylene glycol, maltodextrin, fluorescent protein DNA, etc.

In the present invention, the proportion of the yeast cell extract in the *in vitro* cell-free protein synthesis system is not particularly limited. Generally, the content by volume of the yeast cell extract in the *in vitro* cell-free protein synthesis system is in a range of 20%-70%, preferably, in a range of 30%-60%, more preferably, in a range of 40%-50%.

Preferably, the luminescence value of each target protein in Step (3) is not interfered by other proteins at the maximum emission wavelength.

The concentration in the concentration percentage of each target protein in Step (3) refers to the final concentration of each target protein synthesized in the reaction solution after a period of reaction since the template is added in the *in vitro* cell-free protein synthesis system, that is, the concentration of each target protein product. Preferably, the concentration refers to the concentration of each target protein in the reaction solution after the reaction lasts for 16-23 hours.

Preferably, the reaction time in Step (5) is consistent with the reaction time defined in Step (3).

More preferably, the vectors in Step (2) are plasmids containing target protein encoding sequences. That is, the separate vectors containing respective target protein gene are plasmids containing corresponding target protein encoding sequences, respectively.

In another preferred embodiment, the *in vitro* cell-free protein synthesis system in Step (3) is one selected from the group consisting of yeast cell-based *in vitro* protein synthesis system, *Escherichia coli-based in vitro* protein synthesis system, mammal cell-based *in vitro* protein synthesis system, plant cell-based *in vitro* protein synthesis system, insect cell-based *in vitro* protein synthesis system, and combinations thereof.

In another preferred embodiment, the yeast cell is selected from the group consisting of *Saccharomyces cerevisiae, Pichia pastoris* and *Kluyveromyces,* and combinations thereof.

In another preferred embodiment, the luminescence value is relative fluorescence unit (RFU) value.

Preferably, when testing a certain protein to be tested among a plurality of proteins, the luminescence value of the protein to be tested is not interfered by other proteins in the solution when the protein is tested under the conditions of the maximum excitation wavelength and the maximum emission wavelength of the protein and suitable for the use of optical filters.

In some preferred embodiments, the luminescent label is a polyamino acid (having at least 2 amino acid units) with luminescent function, and it can be a peptide or a protein.

In another preferred embodiment, the target protein is a luminescent protein. The luminescent protein is selected from the group consisting of natural luminescent protein, modified luminescent protein and fusion protein containing luminescent protein, and combinations thereof.

In another preferred embodiment, the luminescent protein is a fluorescent protein. The fluorescent protein is selected from the group consisting of natural fluorescent protein, modified fluorescent protein and fusion protein containing fluorescent protein, and combinations thereof.

In another preferred embodiment, the fluorescent protein is red fluorescent protein, orange fluorescent protein, yellow fluorescent protein, green fluorescent protein, cyan fluorescent protein, blue fluorescent protein or purple fluorescent protein.

In another preferred embodiment, the method for quantitatively co-expressing multiple proteins *in vitro* further comprises separation and/or purification of the target proteins, that is, it comprises at least one of the following processes: isolation of the target proteins, and purification of the target proteins.

According to the second aspect, the present invention provides a use of the *in vitro* cell-free protein synthesis system in the method of quantitatively co-expressing multiple proteins *in vitro* according to the first aspect of the invention.

According to the third aspect, the present invention provides one or a plurality of fluorescent proteins expressed by using the *in vitro* cell-free protein synthesis system. Wherein, the plurality of fluorescent proteins are co-expressed in the same reaction system.

The main advantages of the present invention include:

(1) For the first time, the invention provides a method for quantitatively co-expressing multiple proteins *in vitro*. In this method, the multiple proteins are synthesized by using an *in vitro* cell-free protein synthesis system, which is simple, efficient and fast. When it is used to synthesize fluorescent proteins, measurable fluorescence intensity, which is visually detectable with naked eyes, can be generated. Compared with conventional methods, it can monitor expressed proteins in real time in an efficient and intuitive manner, and it allows complex phenomenon to be simplified.

(2) A method for quantitatively co-expressing multiple fluorescent proteins, that is, a method for simultaneously synthesizing multiple proteins in the same system, is provided. In this method, the target proteins can be synthesized quantitatively at a preset ratio.

(3) The method herein can be used to synthesize therapeutic proteins. For example, the method can be configured to quantitatively co-express heavy chain protein and light chain protein of antibodies *in vitro* to synthesize the heavy chain protein and light chain protein of antibodies at a ratio.

**BRIEF DESCRIPTION OF THE DRAWINGS**

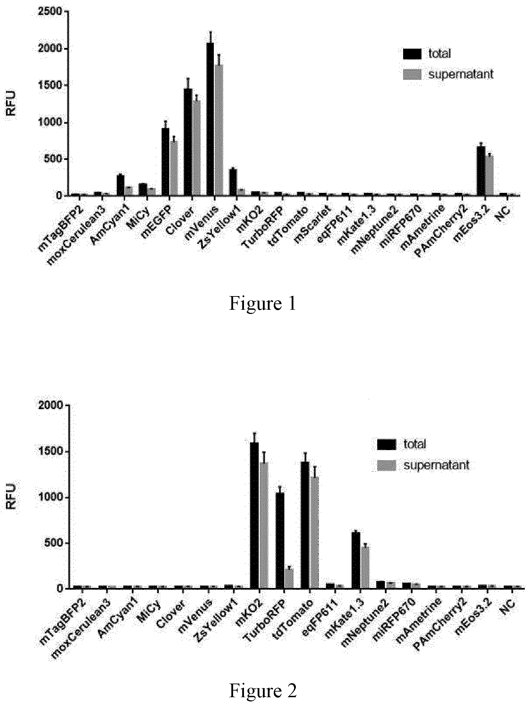

Figure 1 is a graph schematically illustrating the determination of Relative Fluorescence Unit (RFU) value of fluorescent protein synthesized *in vitro* when the maximum Ex/Em (Excitation/Emission wavelength) is 488/507 nm to recognize the green series (containing cyan, green, and yellow). Figure 1 shows total RFU value (i.e., RFU value of the reaction solution after reaction is completed) of multiple fluorescent proteins (including 19 proteins, for example, moxCerulean3, AmCyan1, MiCy, mEGFP, Clover, mVenus, ZsYellow1 and mEos3.2) and the RFU value of the supernatant of the proteins being subjected to centrifugation, wherein the expression level of AmCyan1 and ZsYellow1 in supernatant is relatively lower.

Figure 2 is a graph schematically illustrating the determination of Relative Fluorescence Unit (RFU) value of fluorescent protein synthesized *in vitro* when the maximum Ex/Em (Excitation/Emission wavelength) is 569/593 nm to recognize the red series (containing red, tangerine and far red). Figure 2 shows total RFU value (i.e., RFU value of the reaction solution after reaction is completed) of multiple fluorescent proteins (including 17 proteins, for example, mKO2, TurboRFP, tdTomato, eqFP611, mKate1.3, mNeptune2 and miRFP670) and the RFU value of the supernatant of the proteins being subjected to centrifugation, wherein the expression level of TurboRFP in supernatant is relatively lower.

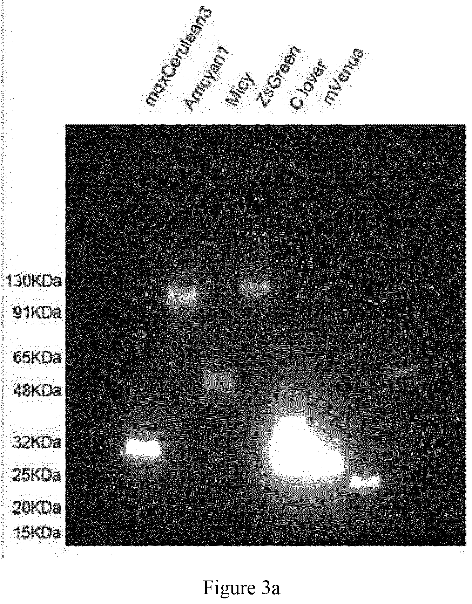

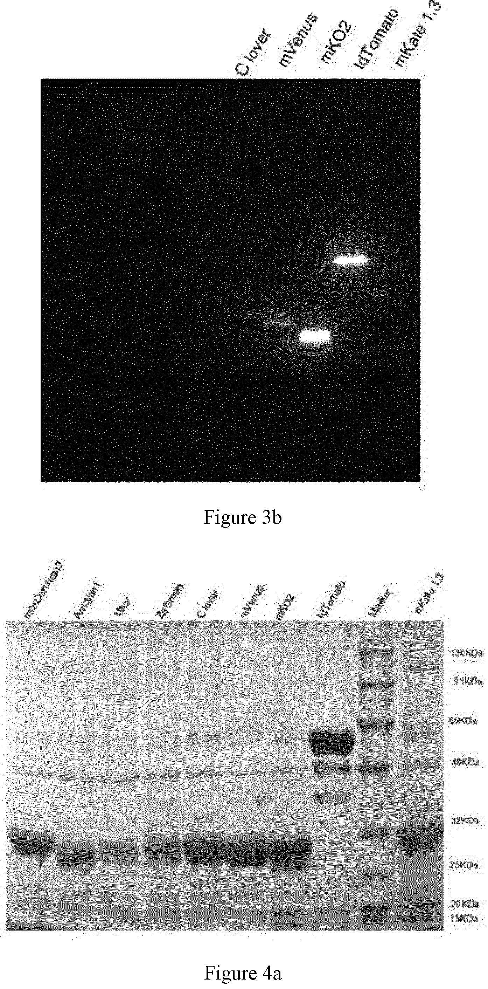

Figure 3 shows results of fluorescence imaging of 9 proteins. Figure 3a shows a fluorescence imaging result when excitation light and emission light have a wavelength of 430 nm and 535 nm, respectively. Figure 3b shows a fluorescence imaging result when excitation light and emission light have a wavelength of 530 nm and 605 nm, respectively. According to the single molecular weight listed in Table 1, together with the molecular weight shown by the SDS-PAGE electrophoresis protein in Figure 3, the aggregate structure of the protein was analyzed. Wherein, AmCyan1 and ZsGreen are tetramer structures, MiCy is a dimer structure, and moxCerulean3, Clover, mVenus, mKO2, tdTomato, and mKate1.3 are monomer structures.

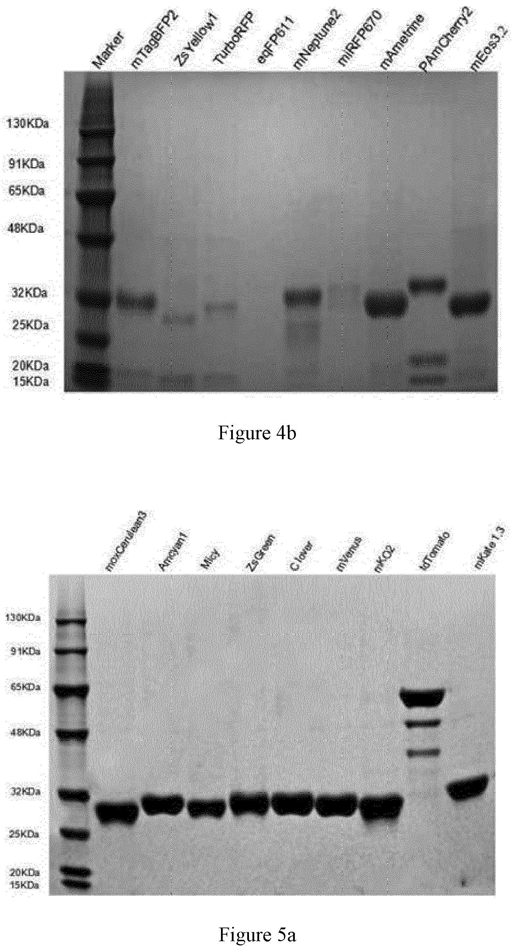

Figure 4 shows the gel imaging results of Coomassiebrilliantblue staining of the purified protein before the optimization of Ni-Beads purification. The results show that only proteins mAmetrine and mEOS3.2 obtain a single high-purity protein, andpurified bandsof AmCyan1, ZsGreen, MiCy, moxCerulean3, Clover, mVenus, mKO2, tdTomato, mKate1.3, mTagBFP2, ZsYellow1, mNeptune2 and PAmCherry are correct and clearly visible, and all contain impurity proteins, wherein miRFP670 band is weak, and eqFP611 does not obtain purified bands.

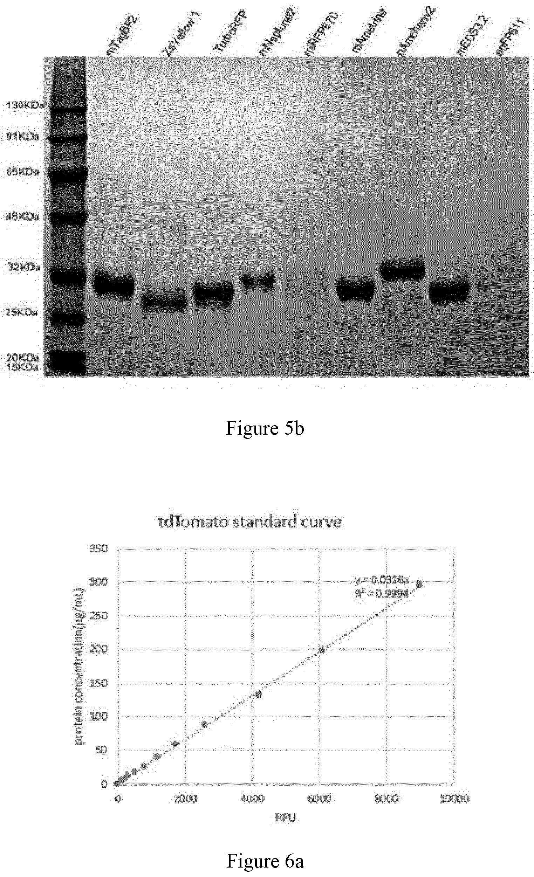

Figure 5 shows the gel imaging results of Coomassiebrilliantblue staining of the purified protein after the optimization of Ni-Beads purification, wherein the purified proteins comprises 18 proteins, namely, AmCyan1, ZsGreen, MiCy, moxCerulean3, Clover, mVenus, mKO2, tdTomato, mKate1.3, mTagBFP2, ZsYellow1, mEOS3.2, TurboRFP, eqFP611, mNeptune2, miRFP670, mAmetrine and PAmCherry, and except for tdTomato, all of the above mentioned proteins obtain single high-purity proteins. The results of Coomassiebrilliantblue staining show that the band size is correct and clearly visible, but the bands of eqFP611 and miRFP670 are weak.

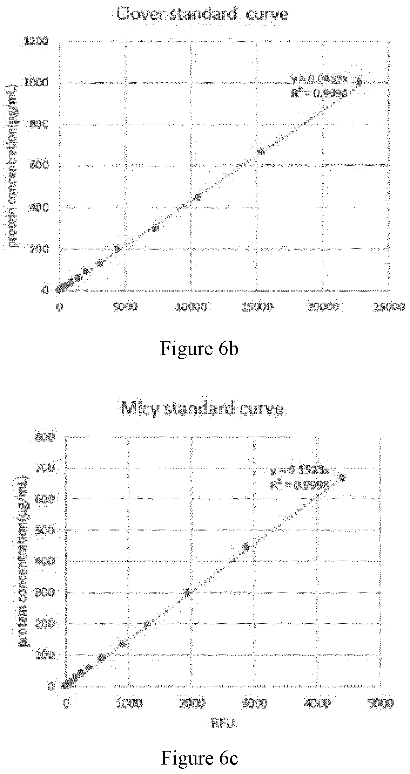

Figure 6 shows the relationship between the concentration of a protein and the relative fluorescence unit (RFU) value. Taking the fluorescent proteins, tdTomato, clover and Micyas examples, the concentration of a single protein is positively correlated with RFUvalue, and the relationship is substantially linear.

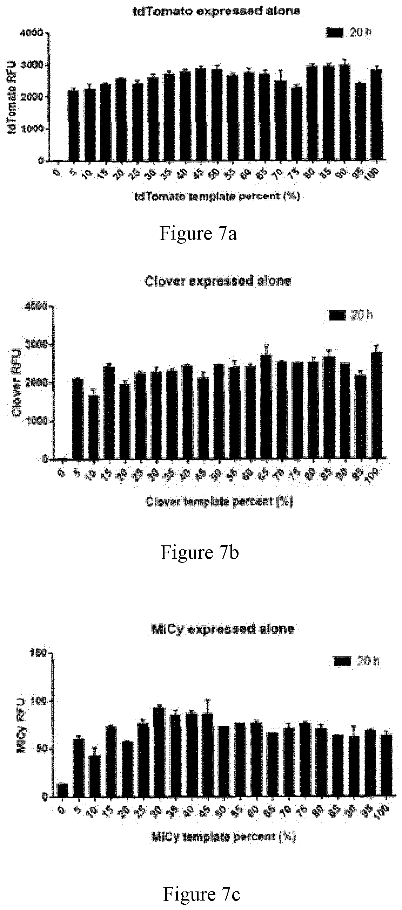

Figure 7 shows the relationship between the DNA templateratio and relative fluorescence unit (RFU) value when a protein is expressed alone. Taking the fluorescent proteins tdTomato, Clover, and Micy as examples, when tdTomato or Clover or MiCy are expressed alone, protein yield is not necessarily associated with the amount of template. The template ratio here refers to a series of different ratios obtained by diluting the concentration of the template solutionto different degrees based on the concentration value at 100% as presented in the figure.

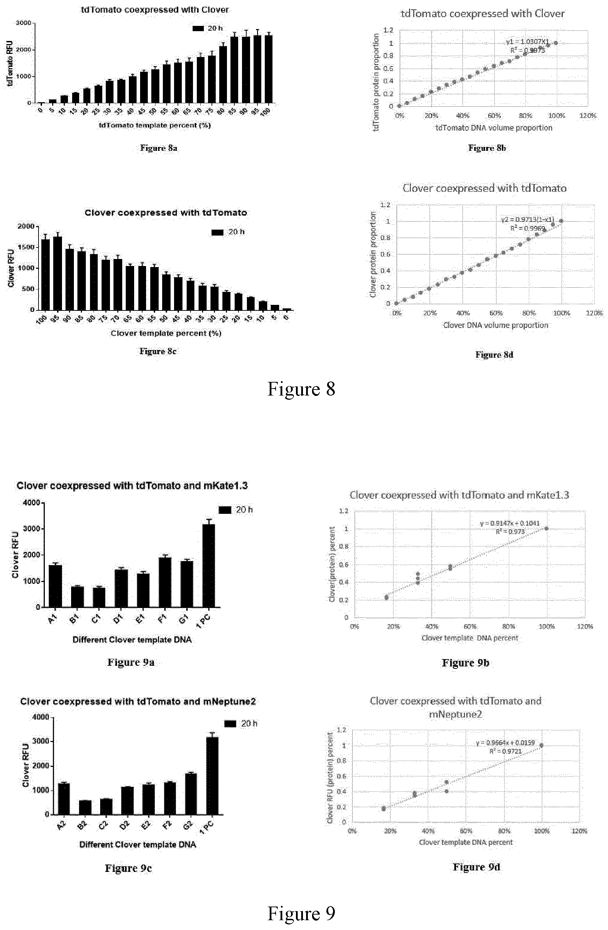

Figure 8 shows the relationship between the template ratio of a protein in a system where two proteins are co-expressed, and relative fluorescence unit (RFU) value. When two proteins are co-expressed, such as tdTomato and Clover are co-expressed in the same reaction system, protein yield is positively correlated with the amount of the template, and the relationship is substantially linear. The template ratio here refers to a series of different ratios of the amount of template of one of the proteinsrelativeto the total amount of templates of the two proteins co-expressed.

Figure 9 shows the relationship between the template ratio of a protein in a system where three proteins are co-expressed, and relative fluorescence unit (RFU) value. When three proteins are co-expressed, such as tdTomato, Clover and mKate1.3 are co-expressed, protein yield is positively correlated with the amount of the template, and the relationship is substantially linear. A1, B1, C1, D1, E1, F1, G1 represent Clover:tdTomato:mKatel.3 template ratiosare 1:1:1, 1:2:3, 1:3:2, 2:1:3, 2:3:1, 3:1:2, 3:2:1, respectively; and A2, B2, C2, D2, E2, F2, G2 represent Clover:tdTomato:mNeptune2 template ratios are 1:1:1, 1:2:3, 1:3:2, 2:1:3, 2:3:1, 3:1:2, 3:2:1, respectively, as shown in Figure 9. The template ratio here refers to a series of different ratios of the amount of template of one of the proteins relativeto the total amount of templates of the three proteins co-expressed.

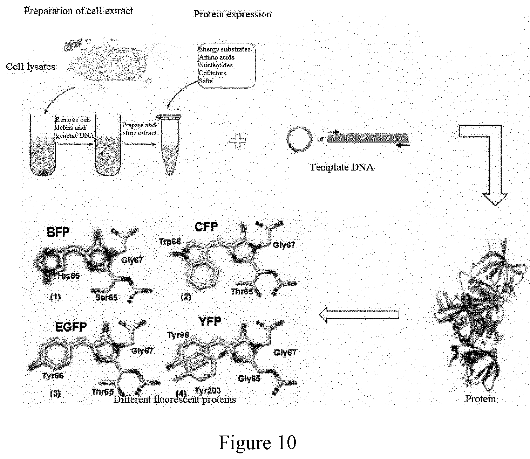

Figure 10 is a flowchart of a process for synthesizing fluorescent proteins. 18 fluorescent proteins are synthesized by using an *in vitro* cell-free protein synthesis system and the synthesized proteins are purified to obtain fluorescent proteins of different colors. The obtained fluorescent proteins have high purity and are visible with naked eyes.

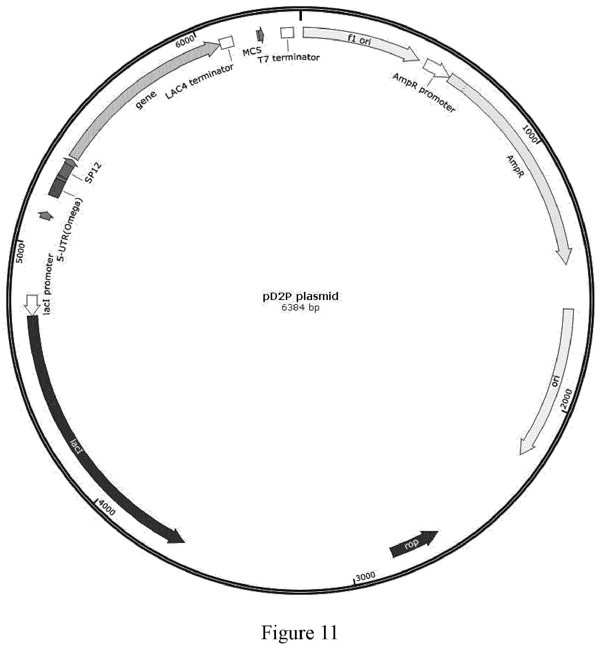

Figure 11 showsa profile of pD2Pplasmid. The pD2P plasmid has a length of 6384 bp, and comprises the following elements: promoter element (not marked in the figure), 5'UTR (including Omega enhancer), signal peptide coding sequence (SP12), target protein coding gene, LAC4 terminator, multiple cloning site (MCS), T7 terminator, replicationorigin (flori), AmpR promoter, ampicillin resistance gene (AmpR gene), high copy number replication origin (ori), gene controlling the copy number of the plasmid (rop gene), lacI promoter, coding sequence of lacI, etc.

## DETAILED DESCRIPTION

### TERMS

As used herein, "*in vitro* protein synthesis system", "*in vitro* cell-free protein synthesis system", "cell-free protein synthesis system" have the same meaning and can be used interchangeably.

As used herein, the term "gene" refers to a nucleotide sequence encoding a certain protein. The term "gene" comprises the coding sequence (CDS) of the protein.

The coding sequence is abbreviated as CDS. The nucleotide sequence completely corresponding to the codon of the protein, and the sequence does not contain other sequences not corresponding to the protein (sequence changes during the process of mRNA processing and other processes are not considered).

As used herein, the expression "having a luminous function" refers to have photosensitivity and it allows to emit light of a detectable wavelength. According to luminescence properties, the emitted light herein include but are not limited to fluorescence, phosphorescence, ultraviolet light, infrared light, and the like. According to the principle of luminescence, the luminous function can be photoluminescence, chemiluminescence, self-luminescence, etc.

In the present invention, the characterization method of substance concentration is not particularly limited, as long as quantification can be achieved by using the following methods, including but not limited to mass concentration, molar concentration, mass volume concentration and volume concentration. For proteins, vectors and other substances, a concentration form suitable for characterization and quantification can be independently used.

As used herein, "concentration of target protein" is preferablymass concentration or mass volume concentration, and other quantifiable concentration forms, such as molar concentration, can also be selected.

As used herein, "multiple" means two or more.

As used herein, "multiple times" means twice or more.

As used herein, "optional" means that it is not necessarily an embodiment of the present invention, but it can be selectively applied depending on the technical solutions of the present invention. Andwhether it is suitable for the technical solutions of the invention is taken as the selection basis.

As used herein, "combinations thereof" means any suitable combinations, including at least two objects listed above.

It should be understood that the above-mentioned technical features of the present invention and the technical features specifically described hereinafter (such as in Examples) can be combined with each other to form a new or preferred technical solution.

**In vitro protein synthesis system**

In some preferred embodiments, the invention provides an *in vitro* protein synthesis system, comprising:

(a) cell extract; preferably, yeast cell extract;
(b) any suitable crowding agent, for example, polyethylene glycol;
(c) optional exogenous sucrose; and
(d) optional solvent, wherein the solvent is water or aqueous solvent.

The wording "optional" incomponents (c) and (d) each independently represents the components (c) and (d) are dispensable.

In a preferred embodiment, the *in vitro* protein synthesis system according to the present invention comprises yeast cell extract, Tris, potassium acetate, magnesium acetate, nucleoside triphosphate mixture (NTPs), amino acid mixture, potassium phosphate, amylase, polyethylene glycol, maltodextrin, etc. Fluorescent protein DNA and other substances can be further added to the *in vitro* protein synthesis system for *in vitro* protein synthesis reactions.

In the present invention, the proportion of the yeast cell extract in the *in vitro*cell-freeprotein synthesis system is not particularly limited. Generally, the content by volume of the yeast cell extract in the *in vitro*cell-freeprotein synthesis system is in a range of20%-70%, preferably, in a range of 30%-60%, more preferably, in a range of 40%-50%.

In the present invention, the cell extract preferably does not contain intact cells. Suitable reported cell extract preparation techniques can be selected to prepare the cell extract. The preparation of the cell extract usually comprises at least the following steps of: providing an appropriate amount of yeast cells, breaking the cells, performing solid-liquid separation, and collecting the supernatant. The extraction product obtained according to the preparation method of the cell extract may have a small or very small amount of intact cells left, and this type of extraction product also falls within the scope of the cell extract of the present invention. The cell extract does not exclude the presence of intact cells.

The *in vitro* protein synthesis system of the present invention also does not exclude the existence of intact cells as long as it does not affect the realization of the purpose of the present invention, that is, it does not affect the realization ofquantitative co-expression. There are many factors behind the presence of those intact cells. The intact cells may be residues caused by the process of preparing the cell extract, or they may be introduced intentionally, for example, the cell fragments obtained by simple fragmentation of cells added may be a mixture of the completely fragmented product and the intact cells; or the intact cellsare present due to the addition of intact cells alone.

Typical cell extract (including yeast cell extract) comprises ribosome, tRNA and aminoacyl tRNAsynthetasefor protein translation, initiation factors, elongation factors and termination release factors required for protein synthesis. Furthermore, the cell extract (including yeast cell extract)also comprises some other proteins derived from the cytoplasm, especially soluble proteins.

In some preferred embodiments, the yeast cell extract is *Kluyveromyces* cell extract. In some preferred embodiments, the *Kluyveromyces* is selected from the group consisting of *Kluyveromyceslactis*(*K.lactis*), *Kluyveromyceslactis var.drosophilarum*, *Kluyveromyceslactis var.lactis,Kluyveromycesmarxianus,Kluyveromycesmarxianus var.lactis,Kluyveromycesmarxianus var.marxianus,Kluyveromycesmarxianus var.vanudenii, Kluyveromycesdobzhanskii,Kluyveromycesaestuarii,Kluyveromycesnonfer     mentans,Kluyveromyceswickerhamii,Kluyveromycesthermotoler-*

*ans,Kluyv eromycesfragilis,Kluyveromyceshubeiensis,Kluyveromycespolysporus,Klu yveromycessiamensis,Kluyveromycesyarrowii,* and combinations thereof.

The protein components (e.g., RNApolymerase)required in the *in vitro* cell-free protein synthesis system can be provided endogenously or be added exogenously. When they are provided endogenously, it is allowed to refer to genetic modification methods provided in the following existing documents and cited documents, including but not limited to: CN108690139A, CN109423496A, CN106978439A, CN110408635A, CN110551700A, CN110093284A, CN110845622A, CN110938649A, CN2018116198190,"Molecular and Cellular Biology, 1990,10(1):353-360". Those methods comprise inserting the coding sequences into an intracellular episomal plasmid, integrating the coding gene into the cell genome, or a combination thereof. When they are provided exogenously, their content can be controlled and adjusted as required by the system.

In some preferred embodiments, the *in vitro* cell-free protein synthesis system comprises:yeast cell extract, Tris, potassium acetate, magnesium acetate, nucleoside triphosphate mixture (NTPs), amino acid mixture, potassium phosphate,sugar (any one of glucose, sucrose, maltodextrin and combinations thereof, and when maltodextrin is contained, amylase is also preferably contained), polyethylene glycol, RNA polymerase, etc. The RNA polymerase can be provided endogenously or added exogenously. One of the more preferred forms of the RNA polymerase is T7 RNA polymerase.

In some preferred embodiments, the *in vitro* cell-free protein synthesis system comprises exogenously added RNA polymerase.

In some preferred embodiments, the *in vitro* cell-free protein synthesis system comprises exogenously added T7 RNA polymerase.

In some preferred embodiments, the *in vitro* cell-free protein synthesis system comprises *Kluyveromyceslactis* cell extract and exogenously added T7 RNA polymerase. In some preferred embodiments, the concentration of the T7 RNA polymerase is in a range of 0.01-0.3 mg/mL. In some other preferred embodiments, the concentration of the T7 RNA polymerase is in a range of 0.02-0.1 mg/mL. In some other preferred embodiments, the concentration of the T7 RNA polymerase is in a range of 0.027-0.054 mg/mL. In some other preferred embodiments, the concentration of the T7 RNA polymerase is 0.04 mg/mL.

In the present invention, the protein content of the yeast cell extract ispreferably in a range of20mg/mL-100 mg/mL, more preferably in a range of 50mg/mL-100 mg/mL. The method for measuring the protein content isCoomassie brilliant blue assay.

The mixture of nucleoside triphosphates in the *in vitro* cell-freeprotein synthesis system preferablycomprises adenosine triphosphate, guanosine triphosphate, cytidine triphosphate and uridine triphosphate. In the present invention, there is no limitation to the concentration of various mononucleotides. Generally, the concentration of each mononucleotide is in a range from 0.5 mM to 5 mM, preferably in a range from 1.0 mM to 2.0 mM.

The amino acid mixture in the *in vitrocell-free* protein synthesis system may comprise natural or unnatural amino acids, and may include amino acidsof D-type or amino acids of L-type. Representative amino acids include, but are not limited to, 20 types of natural amino acids including: glycine, alanine, valine, leucine, isoleucine, phenylalanine, proline, tryptophan, serine, tyrosine, cysteine, methionine, asparagine, glutamine, threonine, aspartic acid, glutamic acid, lysine, arginine and histidine. The concentration of each amino acid is usually in a range from 0.01 mM to 0.5 mM, preferably, in a range from 0.02 mM to 0.2 mM, such as 0.05 mM, 0.06 mM, 0.07 mM and 0.08 mM.

In some preferred embodiments, the *in vitro* cell-free protein synthesis system further comprises polyethylene glycol (PEG) or analogs thereof. The concentration of polyethylene glycol or analogs thereof is not particularly limited. Generally, the concentration (w/v) of polyethylene glycol or analogs thereof is in a range from 0.1% to 8%, preferably, in a range from 0.5% to 4%, more preferably, in a range from 1% to 2%, based on the total volume of the protein synthesis system. Representative examples of PEG include, but are not limited to, PEG3000, PEG8000, PEG6000 and PEG3350. It should be understood that the system according to the present invention may further comprise polyethylene glycol with other various molecular weights (such as PEG 200, 400, 1500, 2000, 4000, 6000, 8000, 10000 and so on).

In some preferred embodiments, the *in vitro* cell-free protein synthesis system further comprises sucrose. The concentration of sucrose is not particularly limited. Generally, the concentration(w/v) of sucrose is in a range from 0.2% to 4%, preferably, in a range from 0.5% to4%, more preferably, in a range from 0.5% to 1%, based on the total volume of the protein synthesis system.

In addition to the yeast extract, some particularly preferred *in vitrocell*-free protein synthesis systems further comprise the following components: 22 mMTris (pH 8), 30-150 mM potassium acetate, 1.0-5.0 mM magnesium acetate, 1.5-4 mM nucleoside triphosphate mixture, 0.08-0.24 mM amino acid mixture, 20-25 mMpotassium phosphate, 0.001-0.005mg/mL amylase, 1%-4% polyethylene glycol, 320-360mM maltodextrin (based on the molar amount of glucose unit), 8-25 ng/$\mu$L fluorescent protein DNA, etc. Furthermore, the total volume of the *in vitrocell*-free protein synthesis system is in a range from 10 $\mu$L to 10000 $\mu$L, preferably, in a range from 15 $\mu$L to 100 uL, preferably 30 $\mu$L. The yeast extract is more preferably *Kluyveromyces* cell extract, and more preferably *Kluyveromyceslactis*cell extract.

In addition to the yeast extract, someparticularly preferred *in vitrocell*-free protein synthesis systems further comprise the following components: 22 mMTris (pH 8), 30-150 mM potassium acetate, 1.0-5.0 mM magnesium acetate, 1.5-4 mM nucleoside triphosphate mixture, 0.08-0.24 mM amino acid mixture, 20-25 mMpotassium phosphate, 0.001-0.005mg/mL amylase, 1%-4% polyethylene glycol, 320-360mM maltodextrin(based on the molar amount of glucose unit), 0.027-0.054 mg/mL T7 RNA polymerase, etc. Those components can be mixed with 8-25 ng/$\mu$L fluorescent protein DNA for *in vitro* protein synthesis reaction. The reaction volume is preferably in a range from 15 $\mu$L to 100 $\mu$L. One of the preferred volumes is 30 $\mu$L.

## Template

In the present invention, the term "template"refers to a nucleic acid template used to direct protein synthesis, which can be mRNA, DNA templateor a combination thereof, and it is preferably a DNA template. It can be linear or circular, and one of the preferred templates is a circular plasmid.

During the process of *in vitro* protein synthesis, the promoter in the template for initiating the synthesis of target protein can be selected from the group consisting of AOD1, MOX, AUG1, AOX1, GAP, FLD1, PEX8, YPT1, LAC4 , PGK, ADH4, AMY1, GAM1, XYL1, XPR2, TEF, RPS7, T7 and any suitable combinations thereof. One of the preferred promoters is T7 promoter.

## No mutual interference

In the present invention, no mutual interference means that when testingone or more proteins to be tested (i.e., target proteins) in a plurality of proteins are measured in a mixed solution containing a plurality of proteins, the luminescence value of other fluorescent proteins or fusion proteins has little or no overlap with the luminescence value of one or more proteins to be tested under the luminescence detection conditions of the experiment, such as, the conditions of the maximum excitation wavelength, the maximum emission wavelength and suitable for the use of optical filters. It should be noted that if the optical filters used do not match the optical properties of the proteins, it may lead to inaccurate characterization of some linear relationships, such as the linear relationship between the protein ratio and the percentage of the corresponding vector in the following embodiments.

It should be noted that under the fluorescence detection conditions of this experiment, if the luminescence values of other fluorescent proteins or fluorescent fusion proteins have a partial overlap with(i.e., interfere with) the luminescence value of one or more proteins to be tested, thetechnical solution in the present inventioncan be implemented. The partial overlap means that under certain fluorescent detection conditions, the measured luminescence signal comprises luminescence signals of proteins to be tested and of other proteins, and such an overlap does not affect the detection of theproteins to be tested by using the technical solution of the present invention.

## Standard proteins

In the present invention, standard proteins refer to protein samples for calibrating the linear relationship between the concentration of one or more target proteins and the luminescence values. The standard proteins can be fluorescent proteins or fluorescent fusion proteins, which are determined according to the light-emitting unit contained in the target protein molecule to be tested. For example, the standard proteins can be the target proteins or the fluorescent proteins (when the target proteins are fusion fluorescent proteins, it means that the target protein molecules are fused withfluorescent proteins) contained in the structure of the target proteins, or luminescent labels. The purity and concentration of the standard protein samples are known or determined before use.

## Quantitatively co-expressing multiple proteins

In the present invention, such a process refers to one in which the product concentration of the multiple proteins in a reaction system is assigned a specific ratio, and the *in vitro* protein synthesis reaction is initiated at this preset concentration ratio, so that products of the multiple target proteins with preset concentration proportion relationship can be obtained; or, the product concentration of the multiple target proteins in the reaction system is assigned a specific value, and the *in vitro* protein synthesis reaction is initiated at this concentration, so that products of the multiple proteins with desired quantitative relationship can be obtained.

## Fluorescent proteins

Shimomura isolated green fluorescent protein (GFP) from jellyfish for the first time, and Chalfie cloned GFP into other species for expression for the first time. Tsien took the lead in elaborating the chemical mechanism of GFP luminescence, and obtained a GFP mutant (GFP-S65T) with greatly enhanced fluorescence intensity and light stability through single point mutation (S65T) technology. Many scientists, represented by Tsien, introduced genetic mutations into GFP to further transform GFP to obtain blue fluorescent protein (BFP, blue FP), cyan fluorescent protein (CFP, Cyan FP), green fluorescent protein (GFP, green FP) and yellow fluorescent protein (YFP, yellow FP). Later, researchers cloned red-shifted fluorescent proteins from corals, sea anemones and other species,which greatly expanded the multicolor imaging applications of fluorescent proteins. In recent years, scientists have skillfully applied light-activated and light-converted fluorescent proteins to high-resolution imaging, breaking the optical diffraction limit and having a resolution of tens of nanometers. It is a revolutionary leap in the history of microscopic imaging technology. Since then, fluorescent proteinshave become a driver for the further development of life science.

In 1962, Qsamu Shimomura first discovered green fluorescent protein (GFP) in Aequorea Victoria, a jellyfish living in the icy waters of the Arctic Ocean, and isolated and purified GFP. Martin Chalfie discovered the values of GFP, and carried out an experimental research by using GFP, a magic tool for the first time. In 1994, YongjianQian modified GFP, which made the fluorescence of GFP become stronger and change color. Those three scientists won the Nobel Prize in Chemistry in 2008. Since then, fluorescent proteinshaveled to a new revolution in biotechnology. The GFP found in jellyfish is composed of 238 amino acids with a molecular weight of 26.9kDa, and amino acids at positions 65, 66, and 67 spontaneously form a fluorescent luminescent group --p-hydroxybenzyl imidazolidinone, which can be excited by light to produce fluorescence. Many scientists used the luminescence mechanism of fluorescent proteins to extract the fluorescent protein gene from jellyfish and transfer it to other organisms, making biological changes more diversified. Since GFP was cloned in 1992, scientific researchers have designed many GFP mutants and non-mutant proteins, providing powerful research means for modern biological research.

Since the fluorescent proteins have a variety of colors, and their fluorescence is stable and non-toxic, they can develop colors without addition of substrates and cofactors, which are not limited by species, cell types and locations, and the fluorescent proteins can make the complex system structure visualized and can be detected at regular time and at specific positions, so the fluorescent proteins have been widely used. The reported fluorescent protein spectra are distributed throughout the visible region and are widely used in biological research fields such as gene expression and regulation, protein spatial positioning and transport, protein folding, signal transduction, protease activity analysis, and biomolecular interaction, so there emerge fluorescent mice, fluorescent rabbits, and fluorescent pigs. In the meantime, they are also used in the fields of tumor pathogenesis, drug screening, feed material improvement, aquatic environment detection and nutrition metabolism research, etc.

In the present invention, with consideration of practical use,proteins with different excitation and emission wavelengths, high brightness, different aggregate structures and different colors are selected. A total of 11 types of 18 proteins having the following mutants (proteins modified based on eGFP) characteristics shown in Table 1 are selected.

Table 1 Characteristics of Fluorescent Protein Mutants

| Category | Name | Maximum excitation wavelength (nm) | Maximum emission wavelength (nm) | Brightness | aggregate structure | Extinction Coefficient (l/mol.cm) | Molecular weight (KDa) |
|---|---|---|---|---|---|---|---|
| Blue/UV | mTagBFP2 | 399 | 456 | 32 | Prone to dimerization | 29005 | 29.7 |
| Cyan | moxCerulean3 | 434 | 474 | 36 | Monomer (A206k) | 22920 | 29.8 |
| | AmCyan1 | 453 | 486 | 11 | Tetramer | 26150 | 28.5 |
| | MiCy | 472 | 495 | 25 | Dimer | 26025 | 29.3 |
| Green | ZsGreen | 493 | 505 | 39 | Tetramer | 37400 | 29.3 |
| | Clover | 505 | 515 | 84 | Prone to dimerization | 19035 | 29.8 |
| Yellow | mVenus | 515 | 528 | 53 | Monomer (A206K) | 23505 | 29.9 |
| | ZsYellow 1 | 529 | 539 | 8 | Tetramer | 37400 | 29.3 |
| Orange | mKO2 | 551 | 565 | 40 | Monomer | 26025 | 27.5 |
| | TurboRFP | 553 | 574 | 62 | Dimer | 26150 | 29.3 |
| | tdTomato | 554 | 581 | 95 | Tandem-dimer | 74720 | 57.4 |

(continued)

| Category | Name | Maximum excitation wavelength (nm) | Maximum emission wavelength (nm) | Brightness | aggregate structure | Extinction Coefficient (l/mol.cm) | Molecular weight (KDa) |
|---|---|---|---|---|---|---|---|
| Red | eqFP611 | 559 | 611 | 35 | Tetramer | 24660 | 29.2 |
| Far-red | mKate1.3 | 588 | 635 | 25 | Monomer | 27640 | 29.2 |
| | mNeptune2 | 600 | 650 | 21 | Prone to dimerization | 27765 | 30.6 |
| Near-Infrared | miRFP670 | 642 | 670 | 12 | Monomer | 18045 | 37.7 |
| Long Stokes Shift | mAmetrine | 406 | 526 | 26 | Monomer (A206K) | 24535 | 30 |
| Photoactivable | PAmCherry 2 | 570 | 596 | 13 | Monomer | 34380 | 29.9 |
| Photoconvertible | mEos3.2 | 507/572 | 516/580 | 53/18 | Monomer | 27515 | 28.7 |

**Other citations**

An reported *in vitro* cell-free protein synthesis system, based on the following cell types of *Escherichia coli,* wheat germ cell, rabbit reticulocyte lysate (RRL), *Saccharomyces cerevisiae, Pichia pastoris, Kluyveromycesmarxianus* and other cell types, can be incorporated into the present invention as an alternative form of the *in vitro* protein synthesis system of the present invention. For example, *Escherichia* coli-based *in vitro* cell-free protein synthesis system recorded in WO2016005982A1, and the *in vitro* cell-free protein synthesis system recorded in the cited document in sections, including but not limited to "2.1 Systems and Advantages" on Pages 27-28 in the reference "Lu, Y. Advances in Cell-Free Biosynthetic Technology. Current Developments in Biotechnology and Bioengineering, 2019, Chapter 2, 23-45", can be used to implement thein *vitro* protein synthesis system of the present invention when appropriate.

The *in vitro* protein synthesis system, templates, plasmids, target proteins, *in vitro* protein synthesis reaction (incubation reaction), various preparation methods, various detection methods and other technical elements of the present invention can independently obtain suitable implementation methods from the following documents, including but not limited to: CN106978349A, CN108535489A, CN108690139A, CN108949801A, CN108642076A, CN109022478A, CN109423496A, CN109423497A, CN109423509A, CN109837293A, CN109971783A, CN109988801A, CN109971775A, CN110093284A, CN11048635A, CN110408636A, CN110551745A, CN110551700A, CN110551785A, CN110819647A(CN201808881848), CN110845622A(CN201809550734), CN110938649A (CN2018111131300), CN110964736A(CN2018111423277), CN2018110683534, CN2018116198186, CN2018116198190, CN201902128619, CN2019102355148, CN2019107298813, CN2019112066163, CN2018112862093, CN2019114181518, CN2020100693833, CN2020101796894, CN20201026933X, CN2020102693382, CN2020103469030. Unless they conflict with the purposes of the present invention, these documents and their cited documents are cited herein in their entirety.

The present invention further discloses amethod for quantitatively co-expressing multiple proteins *in vitro,* comprising the steps of:

Step 1, determining the multiple target proteins to be co-expressed and the target expression percentage of each target protein; providing an *in vitro* cell-free protein synthesis system;

Step 2, creating vectors containing respective target protein genes, respectively, for expressing each target protein, respectively; a vector contains only the coding sequence of one target protein;

Step 3, establishing an equation of quantitative relationship between expression percentage of each of the target proteins and percentage of amount of a corresponding vector;

wherein, the vectors of the respective target proteins are added to the *in vitro* cell-free protein synthesis system according to a certain total vector concentration and a certain amount ratio, for *in vitro* protein synthesis reaction; after a specified reaction time, the expression level of each target protein product is measured; the *in vitro* protein synthesis reaction is carried out multiple times according to the preset total vector concentration and a series of different vector amount ratios until it is efficient for analysis and allowed to obtain the equation of quantitative relationship between percentage of expression level of each of the target protein product and the percentage of amount of the corresponding vector by fitting;

Step 4, calculating the percentage of amount of the vectors required for quantitatively co-expressing the multipleproteins;

according to the ratio of the target expression level of the multiple target proteinsto be expressed, the amount of vectors or the amount ratio of the vectors necessary for each of the plurality of target proteins to be expressed is obtained by using the equation established in Step 3 under the condition that the total vector concentration is calculated;

Step 5, quantitatively co-expressing the plurality of target proteins;

according to the amount of vectors or the amount percentage of the vectors necessary for the plurality of target proteins to be expressed, acorresponding amount of the vector of each of the target proteins is added to the *in vitro* cell-free protein synthesis system as described in Step 3for *in vitro* protein synthesis reaction, and the multiple target proteinsco-expressed are obtained after being reacted for the specific period of time defined in Step (3).

Under the guidance of the present invention, a method for quantitatively co-expressing the multiple proteins by quantitatively determining the expression level of a protein product in a non-fluorescent manner or a non-luminescent manner is also within the scope of the present invention. The protein expression level is quantitatively characterized by the non-luminescent manner, such as ultraviolet absorption and infrared absorption.

The present invention is further described below in conjunction with specific examples and the accompanying figures 1-11. The technical flow of the process used in Examples 1-4 is shown in Figure 10. It should be understood that these examples are only used to illustrate the present invention and not to limit the scope of the present invention. With respect to the experimental methods without specifically described conditions in the following examples,one person may generally follow conventional conditions, such as the conditions described in Sambrooket al., Molecular Cloning: A Laboratory Manual (New York: Cold Spring Harbor Laboratory Press, 1989), or follow the conditions recommended by the manufacturer. Unless otherwise stated, percentages and portionsrefer to percentages and portions by weight. In the following examples, *Kluyveromyceslactis*(*Kluyveromyceslactis* NRRL Y-1140) is only an example for illustration, and it does not imply that the present invention is only applied to *Kluyveromyceslactis,* instead, it is only used as a specific expression system of the present invention for research; the technical solution in the examples is also applied to other yeast cell-basedin *vitro* protein synthesis system, *Escherichia coli-basedin vitro* protein synthesis system, mammal cell-based *in vitro* protein synthesis system, plant cell-based *in vitro* protein synthesis system, insect cell-based *in vitro* protein synthesis system.

Unless otherwise specified, materials and reagents used in the examples of the present invention are all commercially available products.

**Example 1**

**DNA Screening and Codon Optimization of Different Fluorescent Protein Expression Sequences**

By searching different databases, the coding sequences of 18 different fluorescent protein genes were subjected to Blast, and the codons were optimized to make them suitable for *Kluyveromyceslactis*-based *in vitro* cell-free protein synthesis system.

**Example 2**

**Construction of Plasmids Containing Eukaryotic Translation Regulatory Sequence, Fluorescent Protein Coding Sequence, Tag (Protein Expression and Purification Sequence)for the *in vitro Cell-free* Protein Synthesis System**

2.1 Whole gene synthesis

The optimized DNA sequences in Table 2 were used for genome synthesis.

2.2 Primer design

Primer designing was performed by Oligo 7.0 software, and sequences of primers are shown in Table 2.

Table 2 Primer sequences

| No. | Primer name | Primer sequence (5'to3') |
|---|---|---|
| 1 | vector-FP-F1 | TAAATAAGGATTAATTACTTGGATGCCAAT |

(continued)

| No. | Primer name | Primer sequence (5'to3') |
|-----|-------------|--------------------------|
| 2 | vector-FP-R1 | GCCGCTCCCGTGATGGTGGTGGTGATGGTGGTGTTTCCCACTGTGGGAGAAT |
| 3 | AmCyan1-F1 | CACCACCATCACGGGAGCGGCGCTTTGTCAAATAAGTTCATCGGTGACG |
| 4 | AmCyan1-R1 | CAAGTAATTAATCCTTATTTAGAATGGAACAACTGAAGTAATATGAGCAAC |
| 5 | Clover-F1 | ACCACCATCACGGGAGCGGCGTTTCAAAGGGTGAAGAATTGTTTACTGGT |
| 6 | Clover-R1 | AAGTAATTAATCCTTATTTAATACAATTCATCCATACCATGAGTAATACCAGC |
| 7 | eqFP611-F1 | ACCACCATCACGGGAGCGGCAACTCATTGATCAAGGAAAACATGAGAATGATG |
| 8 | eqFP611-R1 | CCAAGTAATTAATCCTTATTTACAATCTACCCAATTTTGATGGCAAATCAC |
| 9 | mAmetrine-F 1 | CCACCATCACGGGAGCGGCGTTTCTAAGGGTGAAGAATTGTTCACTGGT |
| 10 | mAmetrine-R 1 | AAGTAATTAATCCTTATTTATTTATACAATTCATCCATACCTGGAGTAATACCAGC |
| 11 | mEos3.2-F1 | CCACCACCATCACGGGAGCGGCTCAGCTATTAAGCCAGATATGAAAATTAAGTTGAGG |
| 12 | mEos3.2-R1 | CCAAGTAATTAATCCTTATTTATCTAGCATTATCTGGCAAACCAGAATGAG |
| 13 | MiCy-F1 | CACCACCATCACGGGAGCGGCGTTTCTTACTCTAAGCAAGGTATTGCTCAAGAA |
| 14 | MiCy-R1 | CAAGTAATTAATCCTTATTTATTTAACTTTCAATGGATTAACATGAGCTTCAGCA |
| 15 | miRFP670-F 1 | CACCACCATCACGGGAGCGGCGTTGCTGGTCATGCTTCTGGTT |
| 16 | miRFP670-R 1 | CATCCAAGTAATTAATCCTTATTTAAGATTCCAAAGCAGTAATTCTAGTAGCAATTC |
| 17 | mKate1.3-F1 | CACCACCATCACGGGAGCGGCGTTTCTGAATTGATCAAGGAAAACATGCACATG |
| 18 | mKate1.3-R1 | CCAAGTAATTAATCCTTATTTATCTATGACCCAACTTAGATGGCAAATCACA |
| 19 | mKO2-F1 | CACCACCATCACGGGAGCGGCGTTTCTGTTATCAAGCCAGAAATGAAAATGAG |

(continued)

| No. | Primer name | Primer sequence (5'to3') |
|---|---|---|
| 20 | mKO2-R1 | CAAGTAATTAATCCTTATTTAATGAGCAACAGCATCTTCAACTTGTTC |
| 21 | mNeptune2-F 1 | CCACCACCATCACGGGAGCGGCGTTTCAAAGGGTGAAGAATTGATTAAGG |
| 22 | mNeptune2-R 1 | CCAAGTAATTAATCCTTATTTACTTGTACAATTCATCCATACCATTCAATTTATGACC |
| 23 | moxCerulean 3-F1 | CCACCACCATCACGGGAGCGGCGTTTCTAAGGGTGAAGAATTGTTCACTGGT |
| 24 | moxCerulean 3-R1 | CAAGTAATTAATCCTTATTTACTTGTACAATTCATCCATACCCAAAGTAATACC |
| 25 | mTagBFP2-F 1 | ACCACCATCACGGGAGCGGCGTTTCAAAGGGTGAAGAATTGATTAAGGAAAATATGC |
| 26 | mTagBFP2-R 1 | CCAAGTAATTAATCCTTATTTACAACTTATGACCCAATTTTGATGGC |
| 27 | mVenus-F1 | CACCACCACCATCACGGGAGCGGCGTTTCTAAGGGTGAAGAATTGTTTACTGGTG |
| 28 | mVenus-R1 | CAAGTAATTAATCCTTATTTAGTACAATTCATCCATACCCAAAGTAATACCAGC |
| 29 | PAmCherry2-F1 | ACCACCATCACGGGAGCGGCGTTTCTAAGGGTGAAGAAGATAATATGGCTATTATTAAG |
| 30 | PAmCherry2-R1 | CCAAGTAATTAATCCTTATTTACTTATACAATTCATCCATACCACCAGTTGAATG |
| 31 | tdTomato-F1 | CCACCACCATCACGGGAGCGGCGTTTCAAAGGGTGAAGAAGTTATTAAGGAG |
| 32 | tdTomato-R1 | CCAAGTAATTAATCCTTATTTATTTGTACAATTCATCCATACCATACAAGAACAAATG |
| 33 | TurboRFP-F1 | CACCACCATCACGGGAGCGGCTCAGAATTGATCAAGGAAAACATGCACATG |
| 34 | TurboRFP-R1 | CAAGTAATTAATCCTTATTTATCTATGACCCAATTTAGATGGCAAATCAC |
| 35 | ZsGreen-F1 | CCACCATCACCACCACCATCACGGGAGCGGCGCTCAATCAAAACATGGTTTGACTAAGG |
| 36 | ZsGreen-R1 | GGCATCCAAGTAATTAATCCTTATTTATGGCAAAGCTGAACCAGAAGC |
| 37 | ZsYellow1-1 | CACCACCATCACGGGAGCGGCGCTCATTCTAAGCATGGTTTGAAGGAAG |

(continued)

| No. | Primer name | Primer sequence (5'to3') |
|---|---|---|
| 38 | ZsYellow1-R 1 | CATCCAAGTAATTAATCCTTATTTAAGCCAAAGCTGATGGGAAAGC |

2.3 Construction of plasmids

Inserting gene sequences of target proteins to be expressed into pD2P plasmids (see Figure 11), and construction processof plasmids is as follows:

With pD2P plasmid as template, construction of plasmids was carried out by using molecular cloning techniques. Two PCR amplification processes were carried out by using two pairs of primers, respectively. 8.5 μL product of each PCR amplification process were mixed followed by the addition of 1μL of DpnIand 2 μL of 10 × Cutsmart buffer, and then the mixture was incubated at 37 °C for 3 hours. 5 μL of DpnI-treated productwas added into 50 μL of DH5α competent cells. The mixture was placed on ice for 30 minutes, heat-shocked at 42 °C for 45 seconds, followed by the addition of 1mL of LB liquid medium, and then cultured with shaking at 37 °C for 1 hour. Thereafter, the mixture was coated onto an Amp-resistant LB solid medium, and then an inverted culture was carried out at 37 °C until monoclonal colonies grew out.Three monoclonal colonies were picked out and then cultured for expansion. After it was confirmed to be correct by sequencing, the plasmids were extracted and stored, and the plasmid concentration was adjusted to the same level. Before use, all plasmids were measured based on OD values, and the concentration was adjusted to the same concentration (450ng/μL), that is, the concentration of the mother solution of the template/vector of each target protein is the same.

The coding gene of the target protein in the pD2P plasmid was initiated with a T7 promoter.

**Example 3 Expression of Different Fluorescent Proteins in the Yeast-basedin *vitro*Cell-Free Protein Synthesis System**

All the fragments between the transcription initiation sequence 5' UTR and termination sequence 3' UTR in all the plasmids were amplified with the above-mentioned plasmid as template by using random seven-base primers according to a method for performing amplification process using phi29 DNA polymerase. The amplified products were used as DNA templates for synthesis of various fluorescent proteins. One or more tandem combinations were included between the transcription initiation sequence 5' UTR and terminationsequence 3' UTR. The tandem combination comprises translation-enhancing regulatory elements and protein expression and purification tag elements.

According to the instruction, the prepared DNA templates of the fluorescent proteins (the mother solution concentration of the templates of different fluorescent proteins was the same) were added to self-made *Kluyveromyceslactis*-based *in vitro* cell-free protein synthesis system.

The *in vitrocell-free* protein synthesis system (having a total volume of 30 μL) used in the example comprises: *Kluyveromyceslactis*cell extract 50% (v/v), 22 mMTris (pH 8), 90 mM potassium acetate, 4.0 mM magnesium acetate, 3.0 mM nucleoside triphosphate mixture, 0.16mM amino acid mixture, 22 mMpotassium phosphate, 0.003 mg/mL amylase, 3% (w/v)polyethylene glycol (PEG-8000), 340 mM maltodextrin(in glucose unit, equivalent to about 55mg/mL), 0.04mg/mL exogenously added RNA polymerase, and 15 ng/μL fluorescent protein DNA, etc. When the type of fluorescent protein DNA is greater than 1, 15ng/μL here is the total concentration of all the fluorescent protein DNA.

The above-mentioned reaction system was placed in an environment at 22-30°C, and was incubated for about 20 hours. During the reaction process, different fluorescence may be observed and color of the fluorescence gradually looked darker during a certainperiod of time. After the reaction was completed, the reaction system was immediately placed on the Tecan Infinite F200/M200 multifunctional microplate reader. Different optical filters were selected, and corresponding maximum excitation and emission wavelengths were set according to characteristics of the fluorescent proteins to be measured, the value was read, the strength of each fluorescence signal was detected, and the Relative Fluorescence Unit (RFU) value was taken as an active unit, and the results were shown in Figures 1 and 2.

**Example 4 Purification of Fluorescent Protein**

The fluorescent proteins obtained by the reaction wereoptimized and purified by commercially available nickel beads (Sangon, C600033). Please refer to the instructions for the specific purification method. The purity of the purified protein sample was determined to obtain its protein concentration. The purified protein was made into solution. 1μL 5×SDS-loading buffer (without DTT) was added to 1 μL of the solution for SDS-PAGE; then fluorescence imaging was performed. Several proteins with obvious fluorescence were selected as examples, as shown in Figure 3(3a and 3b). 10μL of the above purified protein was taken out, and 2.5 μL 5×SDS-loading buffer ( containing 500 mM DTT) was added to 10

μL of the purified protein for SDS-PAGE; Coomassie brilliant blue staining, decolorization and gel imaging were performed sequentially; wherein results before optimization were shown in Figure 4(4a and 4b), results after optimization were shown in Figure 5(5a and 5b).

## Example 5 Creating a Standard Curve

1) Detecting the relationship between the concentration of a single protein and the relative fluorescence unit (RFU)

The protein sample purified by nickel beads in Example 4 was used as the standard protein sample. The protein sample was diluted with buffer (500mMNaCl+20mMTris-HCl (pH8.0)) in different gradients way, and the protein of different concentrations was placed on the Tecan Infinite F200/M200 multifunctional microplate reader. Different optical filters were selected, and corresponding maximum excitation and emission wavelengths were set according to characteristics of the fluorescent proteins to be measured, and RFU value was read. The fluorescent proteins tdTomato, clover and Micy were taken as examples, and the concentration of a single protein was positively correlated with RFU value, as shown in Figure 6 (6a-6d). The protein mass concentration standard curves were obtained by fitting the curve by plotting the signal strength against the protein concentration, wherein the protein mass concentration standard curves were as follows:

$$y_1=0.0326X_1, R^2=0.9994$$

$$y_2=0.0433X_2, R^2=0.9994$$

$$y_3=0.1523X_3, R^2=0.9998$$

In the formulas, $y_1, y_2$ and $y_3$ represent the mass concentration (unit: μg/mL) of the proteins tdTomato, Clover and Micy to be tested, respectively; and $X_1$, $X_2$, and $X_3$ represent the luminescence values (RFU) of tdTomato, Clover, and Micy, respectively.

2) Detecting the relationship between the template ratio and the relative fluorescence unit (RFU)value when a single protein was expressed. The prepared DNA templates of the fluorescent proteins (the mother solution concentration of the templates of different fluorescent proteins was the same) were added to self-made *Kluyveromyceslactis*-based *in vitro* cell-free protein synthesis system.

The *in vitrocell-free* protein synthesis system (having a total volume of 30 μL) used in the example comprises: *Kluyveromyceslactis*cell extract 50% (v/v), 22 mMTris (pH 8), 90 mM potassium acetate, 4.0 mM magnesium acetate, 3.0 mM nucleoside triphosphate mixture, 0.16mM amino acid mixture, 22 mMpotassium phosphate, 0.003 mg/mL amylase, 3% (w/v) polyethylene glycol, 340 mM maltodextrin (in glucose unit, equivalent to about 55mg/mL), and 15 ng/μL fluorescent protein DNA, etc.

The above-mentioned reaction system was placed in an environment at 22-30°C, and was incubated for about 20 hours. After the reaction was completed, the reaction system was immediately placed on the Tecan Infinite F200/M200 multifunctional microplate reader. Corresponding maximum excitation and emission wavelengths were set according to characteristics of the fluorescent proteins tested, and RFU value was read. The fluorescent proteinstdTomato, Clover, and Micy were taken as examples. When tdTomato, Clover, or MiCy are expressed separately, the protein yield was independent of the amount of template (1μL template per 30μL system), as shown in Figure 7 (7a-7c).

## Example 6 Quantitatively Co-expression of Two Proteins

Detecting the relationship betweenthe volume percentage (i.e., percentage obtained relative to the total amount of the template DNA of the two fluorescent proteins) of the template NDA of a protein in a system where two proteins were co-expressed and the percentage of the amount of the protein mass (i.e.,the ratio of each target protein mass in the total target protein mass, in percentage). The volume percentage of each protein template here was consistent with the concentration percentage of each protein.

The prepared DNA templates of the fluorescent proteins (the mother solution concentration of the templates of different fluorescent proteins was the same) were added to self-made *Kluyveromyceslactis*-based *in vitro* cell-free protein synthesis system.

The *in vitrocell-free* protein synthesis system (having a total volume of 30 μL) used in the example comprises: *Kluyveromyceslactis*cell extract 50% (v/v), 22 mMTris (pH 8), 90 mM potassium acetate, 4.0 mM magnesium acetate, 3.0 mM nucleoside triphosphate mixture, 0.16mM amino acid mixture, 22 mMpotassium phosphate, 0.003 mg/mL amylase, 3% (w/v)polyethylene glycol,340 mM maltodextrin(in glucose unit, equivalent to about 55mg/mL), and 15 ng/μL fluorescent protein DNA (equivalent to the total concentration of the template), wherein the total volume of the two fluorescent proteins was 1μL.

The above-mentioned reaction system was placed in an environment at 22-30°C, and was incubated for about 20 hours. After the reaction was completed, the reaction system was immediately placed on the Tecan Infinite F200/M200 multifunctional microplate reader. Corresponding maximum excitation and emission wavelengths were set according to characteristics of the fluorescent proteinstested, and RFU value was read. When two proteins are co-expressed, for example, when tdTomatoandClover, or tdTomato and MiCy are co-expressed in the same reaction system, it was found that the protein yield was positively correlated with the amount of the template, and the relationshipwas substantially linear, as shown in Figure 8 (8a-8d). In Figure 8, co-expression of tdTomato and Clover was taken as an example to show that the linear relationship between the proportion of a single protein and the percentage of its vector; in addition, the co-expression of tdTomato and MiCy also showed a similarlinear relationship between the proportion of a protein and the percentage of a corresponding vector.

Creating a standard curve by plotting the volume percentage of the template DNA (i.e., the ratio of a certain template volume to the total template volume, which is numerically equal to the ratio of a certain template concentration to the total template concentration) against the obtained concentration percentage of the protein (i.e., the ratio of each protein in the total system to the total protein) as follows, with the co-expression of tdTomato and Clover as an example, by combining the standard curve of Example 5.

When tdTomato and Clover were co-expressed,

$$y_1 = 1.0371x_1, R^2 = 0.9973 \ (0 < x_1 < 0.96)$$

$$y_2 = 0.9713(1 - x_1) = -0.9713x_1 + 0.9713, R^2 = 0.9969$$

In the formulas, $y_1$ and $y_2$ represent the percentage of proteins tdTomato, Clover, $x_1$represents the volume percentage of the template DNA of tdTomato when two proteins are co-expressed.

## Example 7 Quantitatively Co-expression of Three Proteins

Detecting the relationship between the template ratio of a single protein in a system where three proteins were co-expressed and the relative fluorescence unit (RFU) value.

The prepared DNA templates of the fluorescent proteins (the mother solution concentration of the templates of different fluorescent proteins was the same) were added to self-made *Kluyveromyceslactis*-based *in vitro* cell-free protein synthesis system.

The *in vitro*cell-free protein synthesis system (having a total volume of 30 μL) used in the example comprises: *Kluyveromyceslactis*cell extract 50% (v/v), 22 mMTris (pH 8), 90 mM potassium acetate, 4.0 mM magnesium acetate, 3.0 mM nucleoside triphosphate mixture, 0.16mM amino acid mixture, 22 mMpotassium phosphate, 0.003 mg/mL amylase, 3% (w/v)polyethylene glycol,340 mM maltodextrin(in glucose unit, equivalent to about 55mg/mL), and 15 ng/μL fluorescent protein DNA (equivalent to the total concentration of the template), wherein the total volume of the three fluorescent proteins was 1μL.

The above-mentioned reaction system was placed in an environment at 22-30°C, and was incubated for about 20 hours. After the reaction was completed, the reaction system was immediately placed on the Tecan Infinite F200/M200 multifunctional microplate reader. Corresponding maximum excitation and emission wavelengths were set according to characteristics of the fluorescent proteinstested, and RFU value was read. When three proteins, for example, tdTomato, Clover, and mKate1.3 were co-expressed; and when tdTomato, Clover, and mNeptune2 were co-expressed, it appeared to be a linear relationship similar to that shown in the previous example (i.e., Example 6), that is, protein yield was positively correlated with the amount of the template, and the relationship was substantially linear. A1, B1, C1, D1, E1, F1, G1 represent Clover:tdTomato:mKate1.3 template ratio were1:1:1, 1:2:3, 1:3:2, 2:1:3, 2 :3:1, 3:1:2, 3:2:1, respectively; and A2, B2, C2, D2, E2, F2, G2 represent Clover:tdTomato:mNeptune2template ratio were 1:1:1, 1:2:3, 1:3:2, 2:1:3, 2:3:1, 3:1:2, respectively, as shown in Figure 9 (9a-9d).

The protein percentage standard curves fitted by the template ratio and the protein yield ratioin this example were as follows:

When tdTomato, Clover and mKate1.3 were co-expressed,

$$y_5 = 0.9147x_3 + 0.1041, R^2 = 0.973 \ (0 < x_3 < 0.979)$$

In the formula, $y_5$ is the content of the protein Clover to be measured (relative to the total amount of all the proteins), $x_3$ is the volume percentage of the template DNA of Clover when three proteins are co-expressed.

When tdTomato, Clover and mNeptune2were co-expressed,

$$y_6=0.9664x_4+0.0159,\ R^2=0.9721\ (0<x_4<1)$$

In the formula, $y_6$ is the content of the protein Clover to be measured (relative to the total amount of all the proteins), $x_4$ is the volume percentage of the template DNA of Clover when three proteins are co-expressed.

In the Figure 9, only Clover was taken as an example to show that the linear relationship between the proportion of its protein and the percentage of its vector; in addition, the other two proteins co-expressed also showed a similar linear relationship between the proportion of a protein and the percentage of a corresponding vector.

**Example 8 Quantitatively Co-expression of Two Fluorescent Proteins, tdTomato and Clover**

The mass concentration ratio of the two target proteins synthesized was 1:1 as required, that is, the concentration of the tdTomato protein is 50%,and the concentration of the Micy protein was 50%. The volume ratio relationship (consistent with the concentration ratio relationship)of the template DNA of the two fluorescent proteins was calculated according to the equation obtained in Example 6 when tdTomato and Clover were co-expressed:

$$y_1=1.0371x_1,\ R^2=0.973\ (0<x_1<0.96)$$

In the formula, y represents the percentage of tdTomato protein, $1-y_1$ represents the percentage of Clover protein, and $x_1$ is the volume percentage of tdTomato template DNA when the two proteins are co-expressed.

That is to say, when $y_1$ was 50%, it was calculated that $x_1$=50%/1.0371=48%, $1-x_1$=1-48%=52%, that is, the volume ratio of the vectors of the two proteins tdTomato and Clover was 0.48:0.52. When the total volume of the two protein vectorswas 1$\mu$L, the volume of the tdTomato vector added therein was0.48 pL, and the volume of the Clover vector added therein was 0.52 $\mu$L.

According to the above calculation results,0.48$\mu$L and 0.52$\mu$L of DNA templates (450ng/$\mu$L) of the two fluorescent proteins, tdTomato and Clover, having the same template mother solution concentration, were added to self-made *Kluyveromyceslactis*-based *in vitro* cell-free protein synthesis system (having a total volume of 30$\mu$L). Wherein the *in vitro* cell-free protein synthesis system comprises *Kluyveromyceslactis* cell extract 50% (v/v), 22 mMTris (pH 8), 90 mM potassium acetate, 4.0 mM magnesium acetate, 3.0 mM nucleoside triphosphate mixture, 0.16mM amino acid mixture, 22 mMpotassium phosphate, 0.003 mg/mL amylase, 3% (w/v)polyethylene glycol,340 mM maltodextrin(in glucose unit, equivalent to about 55mg/mL), and 15 ng/$\mu$L fluorescent protein DNA (equivalent to the total concentration of the template), wherein the total volume of templates of the two fluorescent proteins, tdTomato and Clover was 1 $\mu$L.

The above-mentioned reaction system was placed in an environment at 22-30°C, and was incubated for about 20 hours. After the reaction was completed, the reaction system was immediately placed on the Tecan Infinite F200/M200 multifunctional microplate reader. Corresponding maximum excitation and emission wavelengths were set according to characteristics of the fluorescent proteinstested, and RFU value was read. RFU values of tdTomato and Clover were 1308 and 975, respectively. Substituting the obtained RFU values into the standard curve relational expression described in Example 5:

$$y_1=0.0326X_1,\ R^2=0.9994$$

$$y_2=0.0433X_2,\ R^2=0.9994$$

**In** the formulas, $y_1$ and$y_2$ represent the mass concentration (unit: $\mu$g/mL) of the proteins tdTomatoand Clover to be measured, respectively;and $X_1$, $X_2$represent the luminescence values (RFU) of tdTomato and Clover. It can be obtained from the above formulas that the mass concentration of tdTomato and Clover to be tested were 42.64$\mu$g/mL and 42.22$\mu$g/mL, respectively, and that the ratio of the mass concentrations of the two proteins was42.64:42.22, similar to 1:1. Such results were substantially the same as we had expected. As a result, the method of the present invention is accurate and feasible for quantitatively co-expressing multiple proteins *in vitro.*

For the first time, the invention provides a method for quantitatively co-expressing multiple proteins *in vitro.*In this method, the multiple proteins are synthesized by using an *in vitro* cell-free protein synthesis system, which is simple, efficient and fast. When it is used to synthesize fluorescent proteins, measurable fluorescence intensity, which is visually detectable with naked eyes, can be generated. Compared with conventional methods, it can monitor expression proteins in real time in an efficient and intuitive manner, and it allows complex phenomenon to be simplified. A method for quantitatively co-expressing multiple fluorescent proteins, that is, a method for simultaneously synthesizing multiple proteins in the same system, is provided. In this method, according to the preset ratio (target ratio), the multiple target proteins can be synthesized quantitatively at thetarget ratio.

The above descriptions are only part of embodiments of the present invention;the invention is not limited to those embodiments. Under the guidance and teachings of the technical solution of the present invention, many modifications and variations having the same technical effects will be apparent to those of ordinary sill in the art and are within the scope of the present invention.

All documents mentioned in the present invention are cited as references in this application, just as each document is individually cited as a reference. Additionally, it should be understood that those skilled in the art can make various changes or modifications to the present invention in light of the above teachings, and the equivalents also fall into the scope as defined by the appended claims of this application.

**References:**

1.Shimomura O, Johnson FH, Saiga Y. Extraction,purification and properties of aequorin, a bioluminescent protein from the luminous hydromedusan, Aequorea. J Cell Comp Physiol,1962; 59: 223-239.

2. Smith M T, Wilding K M, Hunt J M, et al. The emerging age of cell-free synthetic biology[J]. FEBS letters, 2014, 588(17): 2755-2761.

3.Heim R, Cubitt A, Tsien RY. Improved green fluore scene. Nature, 1995, 373: 663-664.

4.Shaner NC, Campbell RE, Steinbach PA, GiepmansBN,Palmer AE, Tsien RY. Improved monomeric red, orange and yellow fluorescent proteins derived from Discosoma sp. red fluorescent protein. Nat Biotechnol, 2004, 22:1567-1572.

5.Gurskaya, N.G. et al. GFP-like chromoproteins as a source of far-red fluorescent proteins. FEBS Lett, 2001, 507: 16-20.

6.Nagai T, Ibata K, Park ES, Kubota M, Mikoshiba K, Miyawaki AA. Variant of yellow fluorescent protein with fast and efficient maturation for cell-biological applications. Nat Biotechnol, 2002, 20: 87-90.

7.Chong, S., Overview of Cell-Free Protein Synthesis: Historic Landmarks, Commercial Systems, and Expanding Applications. 2014: John Wiley & Sons, Inc. 16.30.1-16.30.11.

8.Chudakov D M, Lukyanov S, Lukyanov K A. Fluorescent proteins as a toolkit for in vivo imaging[J]. Trends in biotechnology, 2005, 23(12): 605-613.

9.Shaner N C, Patterson G H, Davidson M W. Advances in fluorescent protein technology[J]. Journal of cell science, 2007, 120(24): 4247-4260.

10.Satoshi KARASAWA*, Toshio ARAKI*, Takeharu NAGAI*, Hideaki MIZUNO* and Atsushi MIYAWAKI*. Cyan-emitting and orange-emitting fluorescent proteins as a donor/acceptorpair for fluorescence resonance energy transfer. Biochem.J. (2004) 381, 307-312 (Printed in Great Britain).

11. JIN-HO AHN et al. "Tuning the expression level of recombinant proteins by modulating mRNA stability in a cell-free protein synthesis system", BIOTECHNOLOGY AND BIOENGINEERING, JOHN WILEY, HOBOKEN, USA, vol. 101, no. 2, 7 March 2008 (2008-03-07), pages 422-427, XP071134047, ISSN: 0006-3592, DOI: 10.1002/BIT.21884

Although the specific embodiments of the present invention are described above, those skilled in the art should understand that these are only examples, and many modifications and variations can be made to these embodiments without departing from the principle of the invention. Therefore, the scope of the invention is defined by the appended claims.

**Claims**

1. A method for quantitatively co-expressing multiple proteins *in vitro,* comprising the steps of:

(1) establishing a standard curve:
establishing standard curve of protein concentration-luminescence intensity relationship for each co-expressed protein using corresponding standard protein;
(2) creating separate vectors containing each target protein gene for expressing each target protein respectively;
(3) establishing an equation of quantitative relationship between concentration percentage of each of target proteins and concentration percentage of the corresponding vector;

wherein the separate vectors in Step (2) containing the target protein genes are added at different concentration ratios to *in vitro* cell-free protein synthesis system for protein synthesis reaction; after a specified reaction time, a luminescence value for each target protein in the reaction solution is obtained; concentration of each target protein product is calculated according to the standard curve shown in Step (1), and an equation of quantitative relationship between concentration percentage of each target protein product and concentration percentage of the corresponding vector is obtained by fitting; in *in vitro* cell-free protein synthesis system, the total concentration of the vectors remains the same;

(4) calculating concentration and concentration ratio of the vectors for quantitatively co-expressing multiple proteins;

wherein, according to target concentration ratio relationship of the multiple target proteins to be expressed, the concentration and concentration ratio of the vector for each of the multiple target proteins to be expressed are calculated by using the equation established in Step (3);

(5) quantitatively co-expressing the multiple proteins;

wherein, according to the required concentration or concentration ratio of each target protein vector obtained in Step (4), corresponding amount of the independent vector of each target protein is added to the *in vitro* cell-free protein synthesis system as described in Step (3), and after the specific period of time defined in Step (3), the co-expressed multiple target proteins are obtained;

wherein the multiple target proteins are each independently luminescent protein or fusion protein carrying a luminescent label.

2. A method for quantitatively co-expressing multiple proteins *in vitro,* comprising the steps of:

(1) establishing a standard curve:

establishing standard curve of protein concentration-luminescence intensity relationship for each co-expressed protein using corresponding standard protein;

(2) creating separate vectors and mother solutions thereof containing each target protein gene for expressing each target protein respectively, wherein the concentration of the separate vectors of each target protein in each mother solution is the same;

(3) establishing an equation of quantitative relationship between concentration percentage of each target protein and volume percentage of a corresponding vector;

wherein the separate vectors containing the target protein genes in Step (2) are added to the *in vitro* cell-free protein synthesis system at different volume ratios for protein synthesis reaction *in vitro*; after a specified reaction time, a luminescence value for each target protein in a reaction solution is obtained; the concentration of each target protein product is calculated according to the standard curve shown in Step (1), and the equation of quantitative relationship between concentration percentage of each target protein and the volume percentage of the corresponding vector is obtained by fitting;

in the *in vitro* cell-free protein synthesis system, the total concentration of the vectors remains the same;

(4) calculating the vector volume, and corresponding volume ratio required for quantitatively co-expressing the multiple proteins;

wherein, according to target concentration ratio relationship of the multiple target proteins to be expressed, the volume and volume ratio of the vector required for each of the multiple target proteins to be expressed are calculated by using the equation established in Step (3);

(5) quantitatively co-expressing the multiple proteins;

wherein, according to the required volume and volume ratio for each target protein vector obtained in Step (4), a corresponding amount of the separate vector of each target protein is added to the *in vitro* cell-free protein synthesis system as described in Step (3), and the multiple target proteins co-expressed are obtained after being reacted for the specific period of time defined in Step (3);

wherein the multiple target proteins are each independently luminescent protein or fusion protein carrying a luminescent label.

3. The method for quantitatively co-expressing multiple proteins *in vitro* of claim 1 or claim 2, wherein the luminescence value of each target protein in Step (3) is not interfered by other proteins at a maximum emission wavelength.

4. The method for quantitatively co-expressing multiple proteins *in vitro* of claim 1 or claim 2, wherein the vectors containing respective target protein genes in Step (2) are plasmids containing corresponding target protein encoding sequences, respectively.

5. The method for quantitatively co-expressing multiple proteins *in vitro* of any one of claims 1-4, wherein the *in vitro* cell-free protein synthesis system in Step (3) is one selected from the group consisting of yeast cell-based *in vitro* protein synthesis system, *Escherichia coli*-based *in vitro* protein synthesis system, mammal cell-based *in vitro* protein synthesis system, plant cell-based *in vitro* protein synthesis system, insect cell-based *in vitro* protein synthesis system, and combinations thereof.

6. The method for quantitatively co-expressing multiple proteins *in vitro* of claim 5, wherein the yeast cell is selected from the group consisting of *Saccharomyces cerevisiae*, *Pichia pastoris* and *Kluyveromyces*, and combinations thereof.

7. The method for quantitatively co-expressing multiple proteins *in vitro* of claim 3, wherein the luminescence value is relative fluorescence unit (RFU) value.

8. The method for quantitatively co-expressing multiple proteins *in vitro* of any one of claims 1-7, wherein the luminescent protein is natural fluorescent protein, modified fluorescent protein or fusion protein containing fluorescent protein.

9. The method for quantitatively co-expressing multiple proteins *in vitro* of claim 8, wherein the fluorescent protein is red fluorescent protein, orange fluorescent protein, yellow fluorescent protein, green fluorescent protein, cyan fluorescent protein, blue fluorescent protein or purple fluorescent protein.

10. The method for quantitatively co-expressing multiple proteins *in vitro* of any one of claims 1-9, further comprising isolation and/or purification of the target proteins.

11. A use of an *in vitro* cell-free protein synthesis system in the method of quantitatively co-expressing multiple proteins *in vitro* of any one of claims 1-10.

**Patentansprüche**

1. - Verfahren zum quantitativen Coexprimieren mehrerer Proteine *in vitro,* das die Schritte umfasst:

(1) Erstellen einer Standardkurve:
Erstellen einer Standardkurve für eine Beziehung zwischen Proteinkonzentration und Lumineszenzintensität für jedes coexprimierte Protein unter Verwendung des entsprechenden Standardproteins;
(2) Erzeugen separater Vektoren, die jedes Zielproteingen enthalten, um jedes Zielprotein zu exprimieren;
(3) Erstellen einer Gleichung einer quantitativen Beziehung zwischen einem Konzentrationsprozentsatz jedes Zielproteins und einem Konzentrationsprozentsatz des entsprechenden Vektors;
wobei die separaten Vektoren in Schritt (2), die die Zielproteingene enthalten, in unterschiedlichen Konzentrationsverhältnissen einem System zur zellfreien *In*-vitro-Proteinsynthese für eine Proteinsynthesereaktion zugegeben werden; nach einer bestimmten Reaktionszeit ein Lumineszenzwert für jedes Zielprotein in der Reaktionslösung erhalten wird; eine Konzentration jedes Zielproteinprodukts gemäß der in Schritt (1) dargestellten Standardkurve berechnet wird und eine Gleichung einer quantitativen Beziehung zwischen einem Konzentrationsprozentsatz jedes Zielproteinprodukts und einem Konzentrationsprozentsatz des entsprechenden Vektors durch Anpassung erhalten wird; wobei die Gesamtkonzentration der Vektoren in dem System zur zellfreien In-vitro-Proteinsynthese gleich bleibt;
(4) Berechnen einer Konzentration und eines Konzentrationsverhältnisses der Vektoren zur quantitativen Coexpression mehrerer Proteine;
wobei die Konzentration und das Konzentrationsverhältnis des Vektors für jedes der zu exprimierenden mehreren Zielproteine gemäß einer Zielkonzentrationsverhältnisbeziehung der zu exprimierenden mehreren Zielproteine anhand der in Schritt (3) erstellten Gleichung berechnet werden;
(5) quantitatives Coexprimieren der mehreren Proteine;
wobei eine entsprechende Menge des unabhängigen Vektors jedes Zielproteins gemäß der erforderlichen Konzentration oder dem erforderlichen Konzentrationsverhältnis jedes in Schritt (4) erhaltenen Zielproteinvektors dem System zur zellfreien In-vitro-Proteinsynthese zugegeben wird, wie in Schritt (3) beschrieben, und nach dem in Schritt (3) definierten spezifischen Zeitraum die coexprimierten mehreren Zielproteine erhalten werden; wobei die mehreren Zielproteine jeweils unabhängig lumineszierendes Protein oder Fusionsprotein sind, das eine lumineszierende Markierung trägt.

**2.** - Verfahren zum quantitativen Coexprimieren mehrerer Proteine *in vitro,* das die Schritte umfasst:

(1) Erstellen einer Standardkurve:
Erstellen einer Standardkurve für eine Beziehung zwischen Proteinkonzentration und Lumineszenzintensität für jedes coexprimierte Protein unter Verwendung des entsprechenden Standardproteins;
(2) Erzeugen separater Vektoren und Mutterlösungen davon, die jedes Zielproteingen enthalten, um jedes Zielprotein zu exprimieren, wobei die Konzentration der separaten Vektoren jedes Zielproteins in jeder Mutterlösung gleich ist;
(3) Erstellen einer Gleichung einer quantitativen Beziehung zwischen einem Konzentrationsprozentsatz jedes Zielproteins und einem Volumenprozentsatz eines entsprechenden Vektors;

wobei die separaten Vektoren, die die Zielproteingene in Schritt (2) enthalten, dem System zur zellfreien *In*-vitro-Proteinsynthese in unterschiedlichen Volumenverhältnissen für eine Proteinsynthesereaktion *in vitro* zugegeben werden; nach einer bestimmten Reaktionszeit für jedes Zielprotein in einer Reaktionslösung ein Lumineszenzwert erhalten wird; die Konzentration jedes Zielproteinprodukts gemäß der in Schritt (1) dargestellten Standardkurve berechnet wird und die Gleichung der quantitativen Beziehung zwischen dem Konzentrationsprozentsatz jedes Zielprotein und dem Volumenprozentsatz des entsprechenden Vektors durch Anpassung erhalten wird;
in dem System zur zellfreien In-vitro-Proteinsynthese die Gesamtkonzentration der Vektoren gleich bleibt;

(4) Berechnen des Vektorvolumens und des entsprechenden Volumenverhältnisses, wie für das quantitative Coexprimieren der mehreren Proteine erforderlich;
wobei das Volumen und das Volumenverhältnis des Vektors, wie für jedes der zu exprimierenden mehreren Zielproteine erforderlich, gemäß einer Zielkonzentrationsverhältnisbeziehung der zu exprimierenden mehreren Zielproteine anhand der in Schritt (3) erstellten Gleichung berechnet werden;
(5) quantitatives Coexprimieren der mehreren Proteine;
wobei eine entsprechende Menge des separaten Vektors jedes Zielproteins dem System zur zellfreien *In-vitro*-Proteinsynthese gemäß dem erforderlichen Volumen und Volumenverhältnis für jeden in Schritt (4) erhaltenen Zielproteinvektor zugegeben wird, wie in Schritt (3) beschrieben, und die mehreren coexprimierten Zielproteine erhalten werden, nachdem sie für den in Schritt (3) definierten spezifischen Zeitraum umgesetzt wurden;
wobei die mehreren Zielproteine jeweils unabhängig lumineszierendes Protein oder Fusionsprotein sind, das eine lumineszierende Markierung trägt.

**3.** - Verfahren zum quantitativen Coexprimieren mehrerer Proteine *in vitro* nach Anspruch 1 oder Anspruch 2, wobei der Lumineszenzwert jedes Zielproteins in Schritt (3) bei maximaler Emissionswellenlänge nicht durch andere Proteine gestört wird.

**4.** - Verfahren zum quantitativen Coexprimieren mehrerer Proteine *in vitro* nach Anspruch 1 oder Anspruch 2, wobei die Vektoren, die die jeweiligen Zielproteingene in Schritt (2) enthalten, Plasmide sind, die jeweils entsprechende Zielproteincodierungssequenzen enthalten.

**5.** - Verfahren zum quantitativen Coexprimieren mehrerer Proteine *in vitro* nach einem der Ansprüche 1 bis 4, wobei das System zur zellfreien In-vitro-Proteinsynthese in Schritt (3) eines ist, das aus der Gruppe ausgewählt ist, die aus In-vitro-Proteinsynthesesystem auf der Basis von Hefezellen, In-vitro-Proteinsynthesesystem auf der Basis von *Escherichia coli,* In-vitro-Proteinsynthesesystem auf der Basis von Säugetierzellen, *In-vitro*-Proteinsynthesesystem auf der Basis von Pflanzenzellen, *In*-vitro-Proteinsynthesesystem auf der Basis von Insektenzellen und Kombinationen davon besteht.

**6.** - Verfahren zum quantitativen Coexprimieren mehrerer Proteine *in vitro* nach Anspruch 5, wobei die Hefezelle aus der Gruppe ausgewählt ist, die aus *Saccharomyces cerevisiae, Pichia pastoris* und *Kluyveromyces* und Kombinationen davon besteht.

**7.** - Verfahren zum quantitativen Coexprimieren mehrerer Proteine *in vitro* nach Anspruch 3, wobei der Lumineszenzwert der Wert der relativen Fluoreszenzeinheit (RFU) ist.

**8.** - Verfahren zum quantitativen Coexprimieren mehrerer Proteine *in vitro* nach einem der Ansprüche 1 bis 7, wobei das lumineszierende Protein natürliches fluoreszierendes Protein, modifiziertes fluoreszierendes Protein oder Fusionsprotein ist, das fluoreszierendes Protein enthält.

**9.** - Verfahren zum quantitativen Coexprimieren mehrerer Proteine *in vitro* nach Anspruch 8, wobei das fluoreszierende Protein rot fluoreszierendes Protein, orange fluoreszierendes Protein, gelb fluoreszierendes Protein, grün fluoreszierendes Protein, cyan fluoreszierendes Protein, blau fluoreszierendes Protein oder violett fluoreszierendes Protein ist.

**10.** - Verfahren zum quantitativen Coexprimieren mehrerer Proteine *in vitro* nach einem der Ansprüche 1 bis 9, das ferner das Isolieren und/oder Reinigen der Zielproteine umfasst.

**11.** - Verwenden eines Systems zur zellfreien *In*-vitro-Proteinsynthese in dem Verfahren zum quantitativen Coexprimieren mehrerer Proteine *in vitro* nach einem der Ansprüche 1 bis 10.

**Revendications**

**1.** - Procédé pour co-exprimer quantitativement de multiples protéines *in vitro,* comprenant les étapes suivantes :

(1) établir une courbe d'étalonnage :
établir une courbe d'étalonnage d'une relation concentration de protéine/intensité de luminescence pour chaque protéine co-exprimée à l'aide d'une protéine étalon correspondante ;
(2) créer des vecteurs distincts contenant chaque gène de protéine cible pour exprimer chaque protéine cible respectivement ;
(3) établir une équation d'une relation quantitative entre un pourcentage de concentration de chacune des protéines cibles et un pourcentage de concentration du vecteur correspondant ;
dans lequel les vecteurs distincts de l'étape (2) contenant les gènes de protéine cible sont ajoutés à différents rapports de concentration à un système de synthèse de protéine acellulaire *in vitro* pour une réaction de synthèse de protéine ; après un temps de réaction indiqué, une valeur de luminescence pour chaque protéine cible dans la solution de réaction est obtenue ; une concentration de chaque produit de protéine cible est calculée en fonction de la courbe d'étalonnage présentée à l'étape (1), et une équation de relation quantitative entre un pourcentage de concentration de chaque produit de protéine cible et un pourcentage de concentration du vecteur correspondant est obtenue par ajustement ; dans le système de synthèse de protéine acellulaire *in vitro,* la concentration totale des vecteurs reste la même ;
(4) calculer une concentration et un rapport de concentration des vecteurs pour une co-expression quantitative de multiples protéines ;
dans lequel, en fonction d'une relation de rapport de concentration cible des multiples protéines cibles à exprimer, la concentration et le rapport de concentration du vecteur pour chacune des multiples protéines cibles à exprimer sont calculés à l'aide de l'équation établie à l'étape (3) ;
(5) co-exprimer quantitativement les multiples protéines ;
dans lequel, en fonction de la concentration ou du rapport de concentration requis de chaque vecteur de protéine cible obtenu à l'étape (4), une quantité correspondante du vecteur indépendant de chaque protéine cible est ajoutée au système de synthèse de protéine acellulaire *in vitro* tel que décrit à l'étape (3) et, après la période de temps spécifique définie à l'étape (3), les multiples protéines cibles co-exprimées sont obtenues ;
dans lequel les multiples protéines cibles sont chacune indépendamment une protéine luminescente ou une protéine de fusion portant une étiquette luminescente.

**2.** - Procédé de co-expression quantitative de multiples protéines *in vitro,* comprenant les étapes suivantes :

(1) établir une courbe d'étalonnage :
établir une courbe d'étalonnage d'une relation concentration de protéine/intensité de luminescence pour chaque protéine co-exprimée à l'aide d'une protéine étalon correspondante ;
(2) créer des vecteurs distincts et des solutions-mères associées contenant chaque gène de protéine cible pour exprimer chaque protéine cible respectivement, la concentration des vecteurs distincts de chaque protéine cible dans chaque solution-mère étant la même ;
(3) établir une équation de relation quantitative entre un pourcentage de concentration de chaque protéine cible et un pourcentage volumique d'un vecteur correspondant ;

dans lequel les vecteurs distincts contenant les gènes de protéine cible de l'étape (2) sont ajoutés au système de synthèse de protéine acellulaire *in vitro* à différents rapports volumiques pour une réaction de synthèse de protéine *in vitro* ; après un temps de réaction indiqué, une valeur de luminescence pour chaque protéine cible dans une solution de réaction est obtenue ; la concentration de chaque produit de protéine cible est calculée en fonction de la courbe d'étalonnage présentée à l'étape (1), et l'équation de relation quantitative entre un pourcentage de concentration de chaque protéine cible et un pourcentage volumique du vecteur correspondant est obtenue par ajustement ;

dans le système de synthèse de protéine acellulaire *in vitro,* la concentration totale des vecteurs reste la même ;

(4) calculer le volume de vecteur et un rapport volumique correspondant requis pour une co-expression quantitative des multiples protéines ;

dans lequel, en fonction d'une relation de rapport de concentration cible des multiples protéines cibles à exprimer, le volume et le rapport volumique du vecteur requis pour chacune des multiples protéines cibles à exprimer sont calculés à l'aide de l'équation établie à l'étape (3) ;

(5) co-exprimer quantitativement les multiples protéines ;

dans lequel, en fonction du volume et du rapport volumique requis pour chaque vecteur de protéine cible obtenu à l'étape (4), une quantité correspondante du vecteur distinct de chaque protéine cible est ajoutée au système de synthèse de protéine acellulaire *in vitro* tel que décrit à l'étape (3), et les multiples protéines cibles co-exprimées sont obtenues après avoir réagi pendant la période de temps spécifique définie à l'étape (3) ;

dans lequel les multiples protéines cibles sont chacune indépendamment une protéine luminescente ou une protéine de fusion portant une étiquette luminescente.

3. - Procédé de co-expression quantitative de multiples protéines *in vitro* selon l'une des revendications 1 ou 2, dans lequel la valeur de luminescence de chaque protéine cible à l'étape (3) n'est pas perturbée par d'autres protéines à une longueur d'onde d'émission maximale.

4. - Procédé de co-expression quantitative de multiples protéines *in vitro* selon l'une des revendications 1 ou 2, dans lequel les vecteurs contenant des gènes de protéine cible respectifs à l'étape (2) sont des plasmides contenant des séquences codantes de protéine cible correspondantes, respectivement.

5. - Procédé de co-expression quantitative de multiples protéines *in vitro* selon l'une quelconque des revendications 1 à 4, dans lequel le système de synthèse de protéine acellulaire *in vitro* de l'étape (3) est choisi dans le groupe consistant enun système de synthèse de protéine *in vitro* à base de cellules de levure, un système de synthèse de protéine *in vitro* à base d'*Escherichia coli,* un système de synthèse de protéine *in vitro* à base de cellules de mammifère, un système de synthèse de protéine *in vitro* à base de cellules végétales, un système de synthèse de protéine *in vitro* à base de cellules d'insecte et des combinaisons de ceux-ci.

6. - Procédé de co-expression quantitative de multiples protéines *in vitro* selon la revendication 5, dans lequel la cellule de levure est choisie dans le groupe consistant en *Saccharomyces cerevisiae, Pichia pastoris* et *Kluyveromyces* et de combinaisons de ceux-ci.

7. - Procédé de co-expression quantitative de multiples protéines *in vitro* selon la revendication 3, dans lequel la valeur de luminescence est une valeur d'unité de fluorescence relative (RFU).

8. - Procédé de co-expression quantitative de multiples protéines *in vitro* selon l'une quelconque des revendications 1 à 7, dans lequel la protéine luminescente est une protéine fluorescente naturelle, une protéine fluorescente modifiée ou une protéine de fusion contenant une protéine fluorescente.

9. - Procédé de co-expression quantitative de multiples protéines *in vitro* selon la revendication 8, dans lequel la protéine fluorescente est une protéine fluorescente rouge, une protéine fluorescente orange, une protéine fluorescente jaune, une protéine fluorescente verte, une protéine fluorescente cyan, une protéine fluorescente bleue ou une protéine fluorescente violette.

10. - Procédé de co-expression quantitative de multiples protéines *in vitro* selon l'une quelconque des revendications 1 à 9, comprenant en outre l'isolement et/ou la purification des protéines cibles.

11. - Utilisation d'un système de synthèse de protéine acellulaire *in vitro* dans le procédé de co-expression quantitative de multiples protéines *in vitro* selon l'une quelconque des revendications 1 à 10.

Figure 1

Figure 2

Figure 3a

Figure 3b

Figure 4a

Figure 4b

Figure 5a

Figure 5b

Figure 6a

Figure 6b

Figure 6c

Figure 7a

Figure 7b

Figure 7c

Figure 8

Figure 9

Figure 10

Figure 11

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- CN 201910460987
- CN 108690139 A
- CN 109423496 A
- CN 106978439 A
- CN 110408635 A
- CN 110551700 A
- CN 110093284 A
- CN 110845622 A
- CN 110938649 A
- CN 2018116198190
- WO 2016005982 A1
- CN 106978349 A
- CN 108535489 A
- CN 108949801 A
- CN 108642076 A
- CN 109022478 A
- CN 109423497 A
- CN 109423509 A
- CN 109837293 A
- CN 109971783 A
- CN 109988801 A
- CN 109971775 A
- CN 11048635 A
- CN 110408636 A
- CN 110551745 A
- CN 110551785 A
- CN 110819647 A
- CN 201808881848
- CN 201809550734
- CN 2018111131300
- CN 110964736 A
- CN 2018111423277
- CN 2018110683534
- CN 2018116198186
- CN 201902128619
- CN 2019102355148
- CN 2019107298813
- CN 2019112066163
- CN 2018112862093
- CN 2019114181518
- CN 2020100693833
- CN 2020101796894
- CN 20201026933X
- CN 2020102693382
- CN 2020103469030

## Non-patent literature cited in the description

- *Molecular and Cellular Biology*, 1990, vol. 10 (1), 353-360
- **LU, Y**. 2.1 Systems and Advantages. *Advances in Cell-Free Biosynthetic Technology*, 27-28
- *Current Developments in Biotechnology and Bioengineering*, 2019, 23-45
- **SAMBROOKET**. Molecular Cloning: A Laboratory Manual. Cold Spring Harbor Laboratory Press, 1989
- **SHIMOMURA O ; JOHNSON FH ; SAIGA Y**. Extraction,purification and properties of aequorin, a bioluminescent protein from the luminous hydromedusan, Aequorea. *J Cell Comp Physiol*, 1962, vol. 59, 223-239
- **SMITH M T ; WILDING K M ; HUNT J M et al.** The emerging age of cell-free synthetic biology[J].. *FEBS letters*, 2014, vol. 588 (17), 2755-2761
- **HEIM R ; CUBITT A ; TSIEN RY**. Improved green fluore scene.. *Nature*, 1995, vol. 373, 663-664
- **SHANER NC ; CAMPBELL RE ; STEINBACH PA ; GIEPMANSBN ; PALMER AE ; TSIEN RY**. Improved monomeric red, orange and yellow fluorescent proteins derived from Discosoma sp. red fluorescent protein. *Nat Biotechnol*, 2004, vol. 22, 1567-1572
- **GURSKAYA, N.G. et al.** GFP-like chromoproteins as a source of far-red fluorescent proteins. *FEBS Lett*, 2001, vol. 507, 16-20
- **NAGAI T ; IBATA K ; PARK ES ; KUBOTA M ; MIKOSHIBA K ; MIYAWAKI AA.** Variant of yellow fluorescent protein with fast and efficient maturation for cell-biological applications.. *Nat Biotechnol*, 2002, vol. 20, 87-90
- **CHONG, S.** Overview of Cell-Free Protein Synthesis: Historic Landmarks, Commercial Systems, and Expanding Applications. John Wiley & Sons, Inc., 2014, 1-11
- **CHUDAKOV D M ; LUKYANOV S ; LUKYANOV K A**. Fluorescent proteins as a toolkit for in vivo imaging [J].. *Trends in biotechnology*, 2005, vol. 23 (12), 605-613

- **SHANER N C** ; **PATTERSON G H** ; **DAVIDSON M W.** Advances in fluorescent protein technology[J].. *Journal of cell science*, 2007, vol. 120 (24), 4247-4260
- **SATOSHI KARASAWA** ; **TOSHIO ARAKI** ; **TAKE-HARU NAGAI** ; **HIDEAKI MIZUNO** ; **ATSUSHI MIYAWAKI\***. Cyan-emitting and orange-emitting fluorescent proteins as a donor/acceptorpair for fluorescence resonance energy transfer. *Biochem.J.*, 2004, vol. 381, 307-312
- Tuning the expression level of recombinant proteins by modulating mRNA stability in a cell-free protein synthesis system. **JIN-HO AHN et al.** BIOTECHNOLOGY AND BIOENGINEERING. JOHN WILEY, 07 March 2008, vol. 101, 422-427